(19) Europäisches Patentamt / European Patent Office / Office européen des brevets

(11) **EP 4 474 468 A1**

(12) **EUROPEAN PATENT APPLICATION**
published in accordance with Art. 153(4) EPC

(43) Date of publication:
**11.12.2024 Bulletin 2024/50**

(51) International Patent Classification (IPC):
*C12M 1/00* (2006.01)          *B01J 19/00* (2006.01)
*B81B 1/00* (2006.01)          *G01N 37/00* (2006.01)

(21) Application number: **22924060.1**

(52) Cooperative Patent Classification (CPC):
**B01J 19/00; B81B 1/00; C12M 1/00; G01N 37/00**

(22) Date of filing: **18.11.2022**

(86) International application number:
**PCT/JP2022/042865**

(87) International publication number:
**WO 2023/145208 (03.08.2023 Gazette 2023/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.01.2022 JP 2022013813**
**30.06.2022 JP 2022106721**

(71) Applicant: **Enplas Corporation**
**Kawaguchi-shi, Saitama 332-0034 (JP)**

(72) Inventors:
• **SUZUKI Seiichiro**
  **Kawaguchi-shi, Saitama 332-0034 (JP)**
• **ONO Koichi**
  **Kawaguchi-shi, Saitama 332-0034 (JP)**
• **SUZUKI Yuichi**
  **Kawaguchi-shi, Saitama 332-0034 (JP)**

(74) Representative: **J A Kemp LLP**
**80 Turnmill Street**
**London EC1M 5QU (GB)**

(54) **FLUID HANDLING DEVICE, METHOD FOR MANUFACTURING SAME, AND METHOD FOR INTRODUCING FLOWABLE MEDIUM TO FLUID HANDLING DEVICE**

(57)  Provided is a fluid handling device in which a fluid can fill not only a specific flow path but also a connection flow path branching from the specific flow path. The fluid handling device includes a main flow path, a first flow path, and a first connection flow path connecting the main flow path to the first flow path. The main flow path is a flow path into which a fluid A is introduced to fill the main flow path and the first connection flow path, and when the fluid A introduced into the main flow path flows from the main flow path into the first connection flow path, a Laplace pressure $P_{L1}$ acting at an interface between the fluid A and a fluid B filling the first connection flow path in advance satisfies Condition 1. Condition 1 is $P_C > P_{L1}$, where $P_C$ is the internal pressure in a branching portion of the main flow path branching to the connection flow path, and $P_{L1}$ is the Laplace pressure when the dynamic contact angle $\theta$ of the fluid A introduced from the main flow path into the connection flow path relative to an inner wall of the connection flow path is set to a value less than $\pi$.

FIG.1

EP 4 474 468 A1

**Description**

Technical Field

The present invention relates to a fluid handling device, a method for manufacturing the same, and a method for introducing a flowable medium into the fluid handling device.

Background Art

In recent years, attention has been given to biofunctional chips (also referred to as an "Organ-on-a-chip") in which a blood vessel model is formed on a chip and biological functions are reproduced. In such a biofunctional chip, for example, a flow path for a cancer cell mass and a flow path for a blood vessel model are formed and put in communication with each other via a connection flow path, and as such, a reaction of the blood vessel model to a substance released from a cancer cell is reproduced as an *in vivo* reaction (Non-Patent Document 1).

Citation List

Non-Patent Document

Non-Patent Document 1: Ioannis K. Zervantonakis et al., PNAS, August 21, 2012, vol. 109, no. 34, 13515-13520

Summary of the Invention

Technical Problem

In such a biofunctional chip, as described above, it is necessary to allow the substance released from the cancer cell mass to react with the blood vessel model via the connection flow path. Therefore, for example, a fluid introduced into a specific flow path needs to also flow into the connection flow path that branches from the specific flow path. However, it has not been clear as to what conditions need to be satisfied by the flow paths in the biofunctional chip in order for the fluid introduced into the specific flow path to be able to flow into and fill the connection flow path.
In view of this, an object of the present invention is to provide a fluid handling device in which, for example, a fluid can fill not only a specific flow path but also a connection flow path branching from the specific flow path.

Solution to the Problem

In order to achieve the aforementioned object, a fluid handling device according to an aspect of the present invention includes a main flow path, a first flow path, and a first connection flow path connecting the main flow path to the first flow path,

wherein the main flow path is a flow path into which a fluid A is introduced to fill the main flow path and the first connection flow path, and
when the fluid A introduced into the main flow path flows from the main flow path into the first connection flow path, a Laplace pressure $P_{L1}$ acting at an interface between the fluid A and a fluid B filling the first connection flow path in advance satisfies Condition 1 below,

$$\text{Condition 1: } P_C > P_{L1},$$

$P_C$: an internal pressure in a branching portion of the main flow path branching to the connection flow path, and
$P_{L1}$: a Laplace pressure when a dynamic contact angle $\theta$ of the fluid A introduced from the main flow path into the connection flow path relative to an inner wall of the connection flow path is set to a value less than $\pi$.

A method for designing a fluid handling device according to an aspect of the present invention includes:

a setting step of setting a structure of a plurality of flow paths included in the fluid handling device,
the fluid handling device including a main flow path, a first flow path, and a first connection flow path connecting the main flow path to the first flow path,
wherein the main flow path is a flow path into which a fluid A is introduced to fill the main flow path and the first connection flow path, and

in the setting step, the structure of the flow paths is designed such that, at a time of use of the fluid handling device, when the fluid A introduced into the main flow path flows from the main flow path into the first connection flow path, a Laplace pressure $P_{L1}$ acting at an interface between the fluid A and a fluid B filling the first connection flow path in advance satisfies Condition 1 below,

$$\text{Condition 1: } P_C > P_{L1},$$

$P_C$: an internal pressure in a branching portion of the main flow path branching to the connection flow path, and
$P_{L1}$: a Laplace pressure when a dynamic contact angle $\theta$ of the fluid A introduced from the main flow path into the connection flow path relative to an inner wall of the connection flow path is set to a value less than $\pi$.

A method for manufacturing a fluid handling device according to an aspect of the present invention includes: a setting step of setting a structure of a plurality of flow paths included in the fluid handling device, using the method for designing a fluid handling device of the present invention; and a forming step of forming flow paths having the set structure on a substrate.

An introducing method for introducing a flowable medium into a fluid handling device according to an aspect of the present invention includes:

an introducing step of introducing a fluid into a flow path of the fluid handling device,
the fluid handling device including a main flow path, a first flow path, and a first connection flow path connecting the main flow path to the first flow path,
wherein the main flow path is a flow path into which a fluid A is introduced to fill the main flow path and the first connection flow path, and
in the introducing step, the main flow path and the first connection flow path are filled with the fluid A by introducing the fluid A into the main flow path at a pressure satisfying Condition 1 below,

$$\text{Condition 1: } P_C > P_{L1},$$

$P_C$: an internal pressure in a branching portion of the main flow path branching to the connection flow path, and
$P_{L1}$: a Laplace pressure when a dynamic contact angle $\theta$ of the fluid A introduced from the main flow path into the connection flow path relative to an inner wall of the connection flow path is set to a value less than $\pi$.

Advantageous Effects of Invention

According to the fluid handling device of the present invention, if the pressure relationship of Condition 1 is satisfied, the fluid introduced into the main flow path can fill not only the main flow path but also the first connection flow path that connects the main flow path to the first flow path.

Brief Description of Drawings

[FIG. 1] FIG. 1 is a schematic diagram showing an example of a fluid handling device.
[FIG. 2] FIG. 2 is a plan view of a partial region of the fluid handling device.
[FIG. 3] FIG. 3 is a plan view of a partial region of the fluid handling device.
[FIG. 4] FIG. 4 is a plan view of a partial region of the fluid handling device.
[FIG. 5] FIG. 5 is a plan view of a partial region of the fluid handling device.
[FIG. 6] FIG. 6 is a schematic diagram showing another example of a first connection flow path in the fluid handling device.
[FIG. 7] FIG. 7 is a schematic diagram showing another example of the first connection flow path in the fluid handling device.
[FIG. 8] FIG. 8 is a schematic diagram showing another example of a first flow path in the fluid handling device.
[FIG. 9] FIG. 9 is a schematic diagram showing another example of a flow path structure in the fluid handling device.
[FIG. 10] FIG. 10 is a schematic diagram showing another example of the flow path structure in the fluid handling device.

[FIG. 11] FIG. 11 is a plan view of a partial region of the fluid handling device.

[FIG. 12] FIG. 12 is a schematic diagram showing another example of the fluid handling device, in which (A) is a top view and (B) is a bottom view.

[FIG. 13] FIG. 13 is a diagram in which (A) is a side view of FIG. 12, (B) is a cross-sectional view taken along line IX-IX in FIG. 12, and (C) is a cross-sectional view of a reservoir taken along line X-X in FIG. 12.

[FIG. 14] FIG. 14 is a cross-sectional view showing an example of a state of use of the reservoir in the fluid handling device.

[FIG. 15] FIG. 15 is a top view of another example of the fluid handling device.

Description of Embodiments

[First Fluid Handling Device and Use Thereof]

The present invention may be embodied in modes such as the following.

[1] A fluid handling device includes a main flow path, a first flow path, and a first connection flow path connecting the main flow path to the first flow path,

wherein the main flow path is a flow path into which a fluid A is introduced to fill the main flow path and the first connection flow path, and
when the fluid A introduced into the main flow path flows from the main flow path into the first connection flow path, a Laplace pressure $P_{L1}$ acting at an interface between the fluid A and a fluid B filling the first connection flow path in advance satisfies Condition 1 below,

$$\text{Condition 1: } P_C > P_{L1},$$

$P_C$: an internal pressure in a branching portion of the main flow path branching to the connection flow path, and
$P_{L1}$: a Laplace pressure when a dynamic contact angle $\theta$ of the fluid A introduced from the main flow path into the connection flow path relative to an inner wall of the connection flow path is set to a value less than $\pi$.

[2] The fluid handling device according to [1], wherein when the fluid A introduced into the main flow path flows from the main flow path into the first connection flow path, a Laplace pressure $P_{L2}$ acting at the interface between the fluid A and the fluid B filling the first connection flow path in advance satisfies Condition 2 below,

$$\text{Condition 2: } P_C < P_{L2},$$

$P_C$: an internal pressure in the branching portion of the main flow path branching to the connection flow path, and
$P_{L2}$: a Laplace pressure when the dynamic contact angle $\theta$ of the fluid A introduced from the main flow path into the connection flow path relative to the inner wall of the connection flow path is set to $\pi$.

[3] The fluid handling device according to [1] or [2], wherein a cross-sectional area of a cross-section of the first connection flow path taken in a direction orthogonal to a flow direction of the fluid A at a position on a first flow path side of the first connection flow path is smaller than a cross-sectional area of a cross-section of the first connection flow path taken in the direction orthogonal to the flow direction of the fluid A at a position on a main flow path side of the first connection flow path.

[4] The fluid handling device according to any one of [1] to [3], wherein a cross-section of the first connection flow path taken in a direction orthogonal to a flow direction of the fluid A is shaped as a quadrangle, the first connection flow path has a constant flow path depth, and a flow path width of the first connection flow path decreases from the main flow path side toward the first flow path side.

[5] The fluid handling device according to any one of [1] to [3], wherein a cross-section of the first connection flow path taken in a direction orthogonal to a flow direction of the fluid A is shaped as a quadrangle, the first connection flow path has a constant flow path width, and a flow path depth of the first connection flow path decreases from the main flow path side toward the first flow path side.

[6] The fluid handling device according to any one of [1] to [3], wherein a cross-section of the first connection flow path taken in a direction orthogonal to a flow direction of the fluid A is shaped as a quadrangle, and a flow path depth and a flow path width of the first connection flow path both decrease from the main flow path side toward the first flow path side.

[7] The fluid handling device according to any one of [1] to [6], wherein in the first connection flow path, the Laplace pressure $P_{L1}$ on the main flow path side is smaller than the Laplace pressure $P_{L1}$ on the first flow path side.

[8] The fluid handling device according to any one of [1] to [7], including a plurality of the first connection flow paths.

[9] The fluid handling device according to any one of [1] to [8], wherein the first flow path has an inlet for introduction of a fluid C and an outlet for discharge of the fluid C, and in the first flow path, a flow resistance from the branching portion, which branches to the first connection flow path, to the outlet is smaller than a flow resistance from the inlet to the branching portion.

[10] The fluid handling device according to any one of [1] to [9], wherein in the fluid handling device of the present invention, the fluid A is a liquid or a sol.

[11] The fluid handling device according to any one of [1] to [10], wherein the main flow path is a flow path for introduction of the fluid A containing a cell mass.

[12] The fluid handling device according to any one of [1] to [11], wherein the first flow path is a flow path for introduction of vascular endothelial cells for forming a blood vessel model.

[13] The fluid handling device according to [12], wherein in the fluid handling device of the present invention, the vascular endothelial cells are HUVEC cells.

[14] The fluid handling device according to any one of [1] to [13], further including a second flow path and a second connection flow path connecting the main flow path to the second flow path,

wherein when the fluid A introduced into the main flow path flows from the main flow path into the second connection flow path, the Laplace pressure $P_{L1}$ acting at the interface between the fluid A and the fluid B filling the second connection flow path in advance satisfies Condition 1 below,

$$\text{Condition 1: } P_C > P_{L1},$$

$P_C$: an internal pressure in the branching portion of the main flow path branching to the connection flow path, and
$P_{L1}$: a Laplace pressure when the dynamic contact angle $\theta$ of the fluid A introduced from the main flow path into the connection flow path relative to the inner wall of the connection flow path is set to a value less than $\pi$.

[15] The fluid handling device according to [14], wherein when the fluid A introduced into the main flow path flows from the main flow path into the second connection flow path, the Laplace pressure $P_{L2}$ acting at the interface between the fluid A and the fluid B filling the second connection flow path in advance satisfies Condition 2 below,

$$\text{Condition 2: } P_C < P_{L2},$$

$P_C$: an internal pressure in the branching portion of the main flow path branching to the connection flow path, and
$P_{L2}$: a Laplace pressure when the dynamic contact angle $\theta$ of the fluid A introduced from the main flow path into the connection flow path relative to the inner wall of the connection flow path is set to $\pi$.

[16] The fluid handling device according to [14] or [15], wherein the main flow path is a flow path for introduction of the fluid A containing a cell mass, and the second flow path is a flow path for introduction of a component involved in growth of the cell mass.

[17] The fluid handling device according to any one of [1] to [16], further including:

a reservoir configured to store a liquid (also called "first reservoir" below), and a circulation flow path,
wherein the reservoir is a tank open upward,
the reservoir and the circulation flow path are in communication with each other, and
an inlet at a first end (one end) of the first flow path and an outlet at a second end (another end) of the first flow path are in communication with each other via the reservoir and the circulation flow path outside the first flow path.

[18] The fluid handling device according to [17], wherein the inlet of the first flow path is in communication with the first reservoir and the outlet of the first flow path is in communication with the circulation flow path, or the outlet of the first flow path is in communication with the reservoir and the inlet of the first flow path is in communication with the circulation flow path. In this fluid handling device, for example, the former is preferable.

[19] The fluid handling device according to [17] or [18],

wherein the reservoir has through holes respectively on an upstream side and a downstream side in a flow direction of the liquid in the first flow path,

the through hole on the upstream side is in communication with a first end (one end) of the circulation flow path, and

the through hole on the downstream side is in communication with the inlet of the first flow path.

[20] The fluid handling device according to any one of [17] to [19], wherein the two through holes are provided in a bottom portion of the first reservoir.

[21] The fluid handling device according to any one of [17] to [20], wherein the first reservoir is a tank to which the liquid to flow in the first flow path and a non-affinity solvent not having affinity for the liquid are added at a time of use.

[22] The fluid handling device according to any one of [17] to [21], wherein a second end (another end) of the circulation flow path is directly in communication with the outlet of the first flow path or is indirectly in communication with the outlet of the first flow path.

[23] The fluid handling device according to any one of [17] to [22], further including:

a second reservoir,

wherein the second reservoir is a tank open upward,

the second reservoir is in communication with a second end (another end) of the circulation flow path, and

the second reservoir is in communication with the outlet of the first flow path.

[24] The fluid handling device according to [23],

wherein the second reservoir has through holes respectively on an upstream side and a downstream side in a flow direction of the liquid in the first flow path,

the through hole on the upstream side is in communication with the outlet of the first flow path, and

the through hole on the downstream side is in communication with the first end (one end) of the circulation flow path.

[25] The fluid handling device according to [23] or [24], wherein the two through holes are provided in a bottom portion of the second reservoir.

[26] The fluid handling device according to any one of [23] to [25], wherein the second reservoir is a tank to which the liquid to flow in the first flow path and a non-affinity solvent not having affinity for the liquid are added at a time of use.

[27] The fluid handling device according to any one of [17] to [26], wherein the circulation flow path has an installation region for a liquid sending portion (also called "liquid sending device" below).

[28] The fluid handling device according to [27], wherein the liquid sending portion is a roller pump or a pressure pump.

[29] A method for designing a fluid handling device, including:

a setting step of setting a structure of a plurality of flow paths included in the fluid handling device,

the fluid handling device including a main flow path, a first flow path, and a first connection flow path connecting the main flow path to the first flow path,

wherein the main flow path is a flow path into which a fluid A is introduced to fill the main flow path and the first connection flow path, and

in the setting step, the structure of the flow paths is designed such that, at a time of use of the fluid handling device, when the fluid A introduced into the main flow path flows from the main flow path into the first connection flow path, a Laplace pressure $P_{L1}$ acting at an interface between the fluid A and a fluid B filling the first connection flow path in advance satisfies Condition 1 below,

$$\text{Condition 1: } P_C > P_{L1},$$

$P_C$: an internal pressure in a branching portion of the main flow path branching to the connection flow path, and

$P_{L1}$: a Laplace pressure when a dynamic contact angle $\theta$ of the fluid A introduced from the main flow path into the connection flow path relative to an inner wall of the connection flow path is set to a value less than $\pi$.

[30] The method for designing a fluid handling device according to [29],

wherein in the setting step, the structure of the flow paths is designed such that, at a time of use of the fluid handling device, when the fluid A introduced into the main flow path flows from the main flow path into the first connection flow path, a Laplace pressure $P_{L2}$ acting at the interface between the fluid A and the fluid B filling the first connection flow path in advance satisfies Condition 2 below,

$$\text{Condition 2: } P_C < P_{L2},$$

$P_C$: an internal pressure in the branching portion of the main flow path branching to the connection flow path, and
$P_{L2}$: a Laplace pressure when the dynamic contact angle $\theta$ of the fluid A introduced from the main flow path into the connection flow path relative to the inner wall of the connection flow path is set to $\pi$.

[31] The method for designing a fluid handling device according to [29] or [30], wherein a cross-sectional area of a cross-section of the first connection flow path taken in a direction orthogonal to a flow direction of the fluid A at a position on a first flow path side of the first connection flow path is set smaller than a cross-sectional area of a cross-section of the first connection flow path taken in the direction orthogonal to the flow direction of the fluid A at a position on a main flow path side of the first connection flow path.

[32] The method for designing a fluid handling device according to any one of [29] to [31], wherein a cross-section of the first connection flow path taken in a direction orthogonal to a flow direction of the fluid A is shaped as a quadrangle, the first connection flow path has a constant flow path depth, and a flow path width of the first connection flow path decreases from the main flow path side toward the first flow path side.

[33] The method for designing a fluid handling device according to any one of [29] to [31], wherein a cross-section of the first connection flow path taken in a direction orthogonal to a flow direction of the fluid A is shaped as a quadrangle, the first connection flow path has a constant flow path width, and a flow path depth of the first connection flow path decreases from the main flow path side toward the first flow path side.

[34] The method for designing a fluid handling device according to any one of [29] to [31], wherein a cross-section of the first connection flow path taken in a direction orthogonal to a flow direction of the fluid A is shaped as a quadrangle, and a flow path depth and a flow path width of the first connection flow path both decrease from the main flow path side toward the first flow path side.

[35] The method for designing a fluid handling device according to any one of [29] to [34], wherein in the first connection flow path, the Laplace pressure $P_{L1}$ on the main flow path side is set smaller than the Laplace pressure $P_{L1}$ on the first flow path side.

[36] The method for designing a fluid handling device according to any one of [29] to [35], wherein the fluid handling device includes a plurality of the first connection flow paths.

[37] The method for designing a fluid handling device according to any one of [29] to [36],

wherein the first flow path has an inlet for introduction of a fluid C and an outlet for discharge of the fluid C, and in the first flow path, a flow resistance from the branching portion, which branches to the first connection flow path, to the outlet is set smaller than a flow resistance from the inlet to the branching portion.

[38] The method for designing a fluid handling device according to any one of [29] to [37], wherein the fluid A is a liquid or a sol.

[39] The method for designing a fluid handling device according to any one of [29] to [38], wherein the main flow path is a flow path for introduction of the fluid A containing a cell mass.

[40] The method for designing a fluid handling device according to any one of [29] to [39], wherein the first flow path is a flow path for introduction of vascular endothelial cells for forming a blood vessel model.

[41] The method for designing a fluid handling device according to [40], wherein the vascular endothelial cells are HUVEC cells.

[42] The method for designing a fluid handling device according to any one of [29] to [41],

wherein the fluid handling device further includes a second flow path, and a second connection flow path connecting the main flow path to the second flow path, and
in the setting step, the structure of the flow paths is designed such that, at a time of use of the fluid handling device, when the fluid A introduced into the main flow path flows from the main flow path into the second connection flow path, the Laplace pressure $P_{L1}$ acting at the interface between the fluid A and the fluid B filling the second connection flow path in advance satisfies Condition 1 below,

$$\text{Condition 1: } P_C > P_{L1},$$

$P_C$: an internal pressure in the branching portion of the main flow path branching to the connection flow path, and $P_{L1}$: a Laplace pressure when the dynamic contact angle of the fluid A introduced from the main flow path into the connection flow path relative to the inner wall of the connection flow path is set to a value less than $\pi$.

[43] The method for designing a fluid handling device according to [42], wherein in the setting step, the structure of the flow paths is designed such that, at a time of use of the fluid handling device, when the fluid A introduced into the main flow path flows from the main flow path into the second connection flow path, the Laplace pressure $P_{L2}$ acting at the interface between the fluid A and the fluid B filling the second connection flow path in advance satisfies Condition 2 below,

$$\text{Condition 2: } P_C < P_{L2},$$

,

$P_C$: an internal pressure in the branching portion of the main flow path branching to the connection flow path, and $P_{L2}$: a Laplace pressure when the dynamic contact angle $\theta$ of the fluid A introduced from the main flow path into the connection flow path relative to the inner wall of the connection flow path is set to $\pi$.

[44] The method for designing a fluid handling device according to [42] or [43], wherein the main flow path is a flow path for introduction of the fluid A containing a cell mass, and the second flow path is a flow path for introduction of a component involved in growth of the cell mass.

[45] An introducing method for introducing a flowable medium into a fluid handling device, including:

an introducing step of introducing a fluid into a flow path of the fluid handling device, the fluid handling device including a main flow path, a first flow path, and a first connection flow path connecting the main flow path to the first flow path, wherein the main flow path is a flow path into which a fluid A is introduced to fill the main flow path and the first connection flow path, and in the introducing step, the main flow path and the first connection flow path are filled with the fluid A by introducing the fluid A into the main flow path at a pressure satisfying Condition 1 below,

$$\text{Condition 1: } P_C > P_{L1},$$

$P_C$: an internal pressure in a branching portion of the main flow path branching to the connection flow path, and $P_{L1}$: a Laplace pressure when a dynamic contact angle $\theta$ of the fluid A introduced from the main flow path into the connection flow path relative to an inner wall of the connection flow path is set to a value less than $\pi$.

[46] The introducing method according to [45], wherein in the introducing step, the fluid A is introduced into the main flow path at a pressure satisfying Condition 2 below,

$$\text{Condition 2: } P_C < P_{L2},$$

$P_C$: an internal pressure in the branching portion of the main flow path branching to the connection flow path, and $P_{L2}$: a Laplace pressure when the dynamic contact angle $\theta$ of the fluid A introduced from the main flow path into the connection flow path relative to the inner wall of the connection flow path is set to $\pi$.

[47] The introducing method according to [45] or [46], wherein the fluid A is a liquid or a sol.

[48] The introducing method according to any one of [45] to [47], wherein the main flow path is a flow path for introduction of the fluid A containing a cell mass.

[49] The introducing method according to any one of [45] to [48], wherein the first flow path is a flow path for introduction of vascular endothelial cells for forming a blood vessel model.

[50] The introducing method according to [45] to [49], wherein the fluid handling device further includes a second flow path, and a second connection flow path connecting the main flow path to the second flow path, and in the introducing step, the main flow path, the first connection flow path, and the second connection flow path are filled with the fluid A by introducing the fluid A into the main flow path at a pressure satisfying Condition 1 below,

$$\text{Condition 1: } P_C > P_{L1},$$

$P_C$: an internal pressure in the branching portion of the main flow path branching to the connection flow path, and
$P_{L1}$: a Laplace pressure when the dynamic contact angle of the fluid A introduced from the main flow path into the connection flow path relative to the inner wall of the connection flow path is set to a value less than $\pi$.

[51] The introducing method according to [50], wherein in the introducing step, the fluid A is introduced into the main flow path at a pressure satisfying Condition 2 below,

$$\text{Condition 2: } P_C < P_{L2},$$

$P_C$: an internal pressure in the branching portion of the main flow path branching to the connection flow path, and
$P_{L2}$: a Laplace pressure when the dynamic contact angle $\theta$ of the fluid A introduced from the main flow path into the connection flow path relative to the inner wall of the connection flow path is set to $\pi$.

[52] The introducing method according to [50] or [51], wherein the main flow path is a flow path for introduction of the fluid A containing a cell mass, and the second flow path is a flow path for introduction of a component involved in growth of the cell mass.

[First Fluid Handling Device]

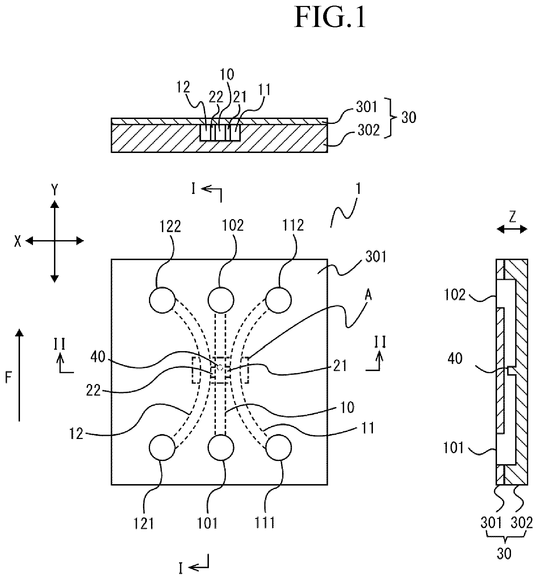

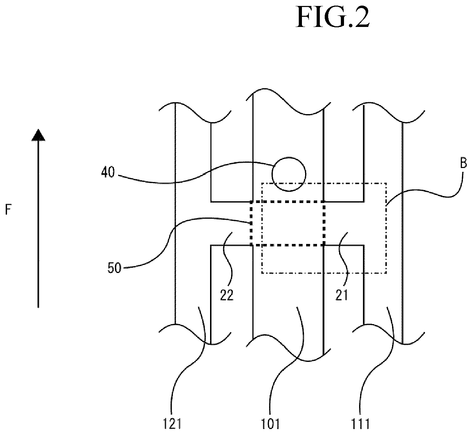

Firstly, in describing the present invention, an example of the basic configuration and the usage of a fluid handling device is shown in FIGS. 1 and 2. FIGS. 1 and 2 are schematic diagrams showing an example of the basic configuration of a fluid handling device, but the fluid handling device of the present invention is not limited in any way to the structure, shape, size, method of use, and the like shown in the drawings.

FIG. 1 is a schematic diagram showing the basic configuration of a fluid handling device 1, in which the lower left portion is a plan view (top view) of the fluid handling device 1 as viewed from the upper surface side, the right portion is a cross-sectional view taken along line I-I, and the upper left portion is a cross-sectional view taken along line II-II. In the present embodiment, in a main flow path 10, a first flow path 11, and a second flow path 12, the side on which a fluid is introduced will be referred to as the upstream side, and the side toward which the introduced fluid flows and is led out (discharged) will be referred to as the downstream side. In FIG. 1, an arrow F indicates the flow direction from the upstream side to the downstream side, and in the plan view of FIG. 1, the lower side is the upstream side of the fluid handling device 1, and the upper side is the downstream side of the fluid handling device 1. The direction connecting the upstream side and the downstream side will be referred to as the Y direction, the direction orthogonal to the Y axis direction in the planar direction of the fluid handling device 1 (also referred to as the vertical direction) will be referred to as the X direction, and the direction orthogonal to both the Y direction and the X direction will be referred to as the Z direction (thickness direction) of the fluid handling device 1. FIG. 2 is an enlarged view of a dashed-dotted line region A in FIG. 1, and for convenience, the dashed lines in FIG. 1 are shown as straight lines in FIG. 2.

The fluid handling device 1 has a body 30 that includes internal flow paths, and the body 30 is a laminate in which an upper substrate 301 and a lower substrate 302 are stacked on one another. The upper surface of the lower substrate 302 has recessed portions, and the upper substrate 301 has through holes. Hollow spaces (internal spaces) that form the main flow path 10, the first flow path 11, and a first connection flow path 21 are formed by the recessed portions of the upper surface of the lower substrate 302 and the lower surface of the upper substrate 301, and further, as shown in FIG. 1, hollow spaces that form the second flow path 12 and a second connection flow path 22 may be formed. For example, a single first connection flow path 21 and a single second connection flow path 22 may be formed as shown in FIG. 1, or more than one of each may be formed as described later. In addition to the main flow path 10, the first flow path 11, and the first connection flow path 21, the fluid handling device of FIG. 1 further includes the second flow path 12 and the second connection flow path 22 as internal flow paths, but in the present invention, the second flow path 12 and the second connection flow path 22 are optional.

The main flow path 10, the first flow path 11, and the second flow path 12 have first ends respectively in communication with inlets 101, 111, and 121, which are in communication with the outside, and also have second ends respectively in communication with outlets (also referred to as discharge ports) 102, 112, and 122, which are also in communication with the outside. The inlets 101, 111, and 121 and the outlets 102, 112, and 122 are formed by through holes provided in the upper substrate 301, and these through holes in the upper substrate 301 respectively face ends of the recessed portions in the upper surface of the lower substrate 302.

The main flow path 10 is in communication with the first flow path 11 via the first connection flow path 21, and is in communication with the second flow path 12 via the second connection flow path 22. The connection flow paths can also be, for example, called slits. In the main flow path 10, a branching region that is in communication with the first connection flow path 21 and the second connection flow path 22 will be referred to as a branching portion 50. A trap portion 40 that protrudes in the thickness direction (Z direction) may be formed inside the main flow path 10 at a position on the downstream side of the branching portion 50.

The fluid handling device 1 may be used as, for example, a biofunctional chip that reproduces an *in vivo* activity between a blood vessel and a mass of cells such as cancer cells. In this case, the fluid handling device 1 can be used as follows.

(1) First, a sol containing a cell mass is introduced into the main flow path 10 such that the main flow path 10, the first connection flow path 21, and the second connection flow path 22 are filled with the sol. The cell mass moves in the flow direction (arrow F) together with the sol and is trapped by the trap portion 40 of the main flow path 10. After the sol is introduced, the sol undergoes conversion into a gel.

(2) Next, a coating agent having affinity for vascular endothelial cells is introduced into, and fills the first flow path 11, thereby coating the inner wall of the first flow path 11.

(3) Then, a culture medium containing vascular endothelial cells is introduced into and fills the first flow path 11 and undergoes cultivation, thereby forming a tubular blood vessel model (also referred to as a pseudo blood vessel) inside the first flow path 11.

In this step, for example, cultivation may be performed while circulating the culture medium in the first flow path 11, or a configuration is possible in which the culture medium is introduced into the first flow path 11 and undergoes cultivation, then the adhesion of vascular endothelial cells to the inner wall of the first flow path 11 is confirmed, then a new culture medium is introduced into the first flow path 11, and then cultivation is performed while circulating the new culture medium.

(4) Next, a fluid containing a component involved in the growth of the cell mass is introduced into, and fills the second flow path 12. The component moves from the second flow path 12 to the main flow path 10 via the second connection flow path 22, thus resulting in a concentration gradient of the component from the second flow path 12 toward the main flow path 10.

(5) The fluid handling device 1 can reproduce a reaction in which, the blood vessel model in the first flow path 11 detects a substance released from the cell mass in the growth process of the cell mass in the presence of the component that has moved from the second flow path 12, and capillaries (also referred to as sprouts or buds of blood vessel branches) are formed in the blood vessel model and extend into the main flow path 10 via the first connection flow path 21. Therefore, if a liquid containing a drug is introduced into the first flow path 11 in which the blood vessel model has been formed, the influence of the drug can be analyzed by checking for the formation or extension of capillaries. In this step, for example, the liquid introduced into the first flow path 11 may be circulated.

In such use of the fluid handling device 1, it is desirable that the sol not only fills the main flow path 10 but also fills the first connection flow path 21 up to the end on the first flow path 11 side. In the case where the second flow path 12 and the second connection flow path 22 are optionally provided, it is further desirable that the sol fills the second connection flow path 22 up to the end on the second flow path 12 side.

In the present invention, by introducing the fluid into the main flow path, the fluid can fill not only the main flow path but also the connection flow path that connects the main flow path to another flow path. Specifically, according to the fluid handling device, the method for manufacturing the fluid handling device, and the method for introducing the fluid into the fluid handling device, the fluid can fill the connection flow path up to the end on the side opposite to the main flow path. Specific examples of the present invention will be described below with reference to the drawings. In the drawings, like portions are denoted by like reference numerals. The present invention is not limited or restricted in any way by the following embodiments. The descriptions of the respective embodiments may be incorporated into one another unless otherwise specified.

(First Embodiment)

A fluid handling device of the present embodiment includes a main flow path, a first flow path, and a first connection flow path connecting the main flow path to the first flow path, wherein the main flow path is a flow path into which a fluid A is introduced to fill the main flow path and the first connection flow path, and when the fluid A introduced into the main flow path flows from the main flow path into the first connection flow path, a Laplace pressure $P_{L1}$ acting at an interface between the fluid A and a fluid B filling the first connection flow path in advance satisfies Condition 1 below.

## Condition 1: $P_C > P_{L1}$,

$P_C$: an internal pressure in a branching portion of the main flow path branching to the connection flow path, and

$P_{L1}$: a Laplace pressure when a dynamic contact angle of the fluid A introduced from the main flow path into the connection flow path relative to an inner wall of the connection flow path is set to a value less than $\pi$.

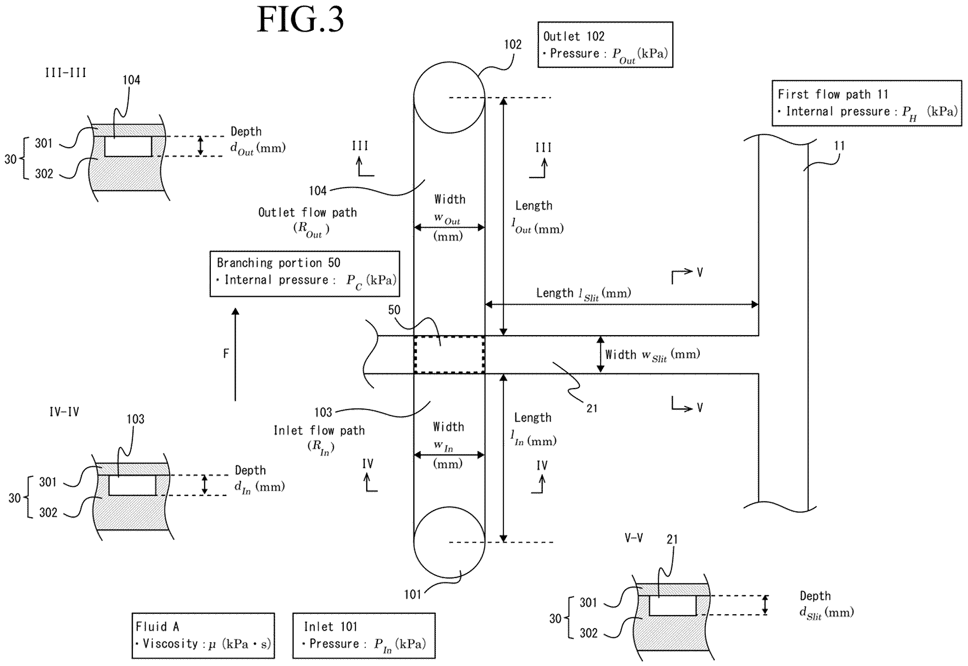

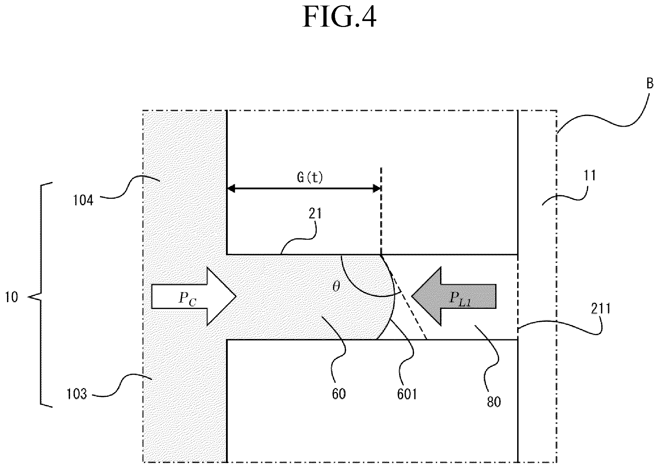

Condition 1 will be described below with reference to the schematic diagrams of FIGS. 3 and 4. FIG. 3 is a schematic diagram showing the main flow path 10, the first flow path 11, and the first connection flow path 21 that connects the main flow path 10 to the first flow path 11, as extracted from the fluid handling device 1 shown in FIGS. 1 and 2. Note that the second flow path 12, the second connection flow path 22 that connects the main flow path 10 to the second flow path 12, and the trap portion 40 are not shown in FIG. 3. In FIG. 3, the inlet 101 side of the main flow path 10 will be referred to as an inlet flow path ($R_{In}$) 103, and the outlet side of the main flow path 10 will be referred to as an outlet flow path ($R_{Out}$) 104. In FIG. 3, the upper right drawing is a plan view showing flow paths, the upper left drawing is a partial cross-sectional view of the fluid handling device 1 taken along line III-III in the plan view, the lower left drawing is a partial cross-sectional view of the fluid handling device 1 taken along line IV-IV in the plan view, and the lower right drawing is a partial cross-sectional view of the fluid handling device 1 taken along line V-V in the plan view. FIG. 3 also shows positions corresponding to the length (1) in the flow direction, the width (w) in the direction orthogonal to the flow direction, the depth (d), and the like of flow paths. In FIG. 3, the length of the inlet flow path ($R_{In}$) 103 of the main flow path 10 is represented by the length from the center of the corresponding inlet 101 to the upstream end of the branching portion 50, and the length of the outlet flow path ($R_{Out}$) 104 of the main flow path 10 is represented by the length from the downstream end of the branching portion 50 to the center of the corresponding outlet 102, but the lengths are not limited to this.

Also, FIG. 4 is an enlarged view of a dashed-dotted line region B in FIG. 2, and shows a state at the time of introducing a fluid A 60 into the main flow path 10. For each flow path, the length is the distance in the direction of flow of the fluid flowing in the flow path, the width is the distance in a direction orthogonal to the direction of flow, and the depth is the distance in a direction orthogonal to the length and the width.

The following specific example is a description of the main flow path 10, the first flow path 11, and the first connection flow path 21 of the fluid handling device 1, but the description similarly applies to the second flow path 12 and the second connection flow path 22 in FIG. 1. In other words, the description of the main flow path 10, the first flow path 11, the first connection flow path 21, and the relationship therebetween in the present embodiment can be applied to the main flow path 10, the second flow path 12, the second connection flow path 22, and the relationship therebetween. Therefore, in Condition 1 regarding the main flow path 10 and the first connection flow path 21, the "connection flow path" in $P_C$ and $P_{L1}$ is the first connection flow path 21 that connects the main flow path 10 to the first flow path 11, and in Condition 1 regarding the main flow path 10 and the second connection flow path 22, the "connection flow path" in $P_C$ and $P_{L1}$ is the second connection flow path 22 that connects the main flow path 10 to the second flow path 12.

In the fluid handling device 1 of the present embodiment, a fluid can be introduced into the flow paths. The fluid will also be referred to as, for example, a flowable medium. In view of this, in the present embodiment, various fluids are referred to as follows for convenience. Specifically, "fluid A" refers to a fluid introduced into the main flow path 10 to fill the main flow path 10 and the first connection flow path 21, and "fluid B" refers to a fluid filling the first connection flow path 21 in advance before the fluid A introduced into the main flow path 10 flows from the main flow path 10 into the first connection flow path 21. The fluid A introduced into the main flow path 10 and the fluid B filling the first connection flow path 21 in advance are different fluids. The different fluids are, for example, fluids that have different viscosities. Specific examples of the fluids will be described later, but for example, the fluid A is a liquid material that has fluidity (including a fluid colloid, for example), and the fluid B is a liquid material different from the fluid A or a gas. The gas is, for example, air.

The fluid introduced into the first flow path 11 and the second flow path 12 will be referred to as "fluid C" for convenience. The fluid C introduced into the first flow path 11 and the fluid C introduced into the second flow path 12 may be, for example, the same or different.

The "branching portion" in the definition of $P_C$ is, for example, a branching region that is in communication with the first connection flow path 21 in the main flow path 10, and is a dashed line region 50 in FIGS. 2 and 3.

"Laplace pressure" generally refers to the pressure difference between the inside and the outside of a fluid across the fluid surface when the surface has a curvature in equilibrium. As described above, the Laplace pressure $P_{L1}$ in Condition 1 is the Laplace pressure acting on the interface between the fluid A (e.g., a liquid material) and the fluid B (e.g., a gas) filling the first connection flow path in advance, when the fluid A introduced into the main flow path flows from the main flow path into the first connection flow path, and the Laplace pressure $P_{L1}$ satisfies Condition 1.

The dynamic contact angle is generally the angle formed by a free surface of a moving fluid in the traveling direction (flow direction) and the surface of a solid in contact with the free surface. The "dynamic contact angle" in $P_{L1}$ in Condition 1 is, for example, a contact angle (advancing contact angle) $\theta$ of a surface 601 (free surface) of the fluid A 60 relative to the inner wall (solid surface) of the first connection flow path 21 in FIG. 4.

As shown in FIG. 4, Pc is the internal pressure in the branching portion 50 of the main flow path 10 that branches to the first connection flow path 21, and is the same in all directions in the branching portion 50. $P_{L1}$ is the Laplace pressure (unit: kPa) when the dynamic contact angle θ of the fluid A 60, flowing from the main flow path 10 into the first connection flow path 21, relative to the inner wall of the first connection flow path 21 is set to a value less than π (rad) (that is to say, when θ<180°). Here, dynamic contact angle θ<π (rad) means that the advancing free surface 601 of the fluid A 60 is moving in the first connection flow path 21 from the end on the main flow path 10 side toward the end on the first flow path 11 side (dashed line 211), that is to say, the free surface has not reached the end on the first flow path 11 side (dashed line 211). In this way, in the fluid handling device 1, due to the Laplace pressure $P_{L1}$ satisfying Condition 1 of "$P_C>P_{L1}$" when the fluid A 60 introduced into the main flow path 10 is introduced into the first connection flow path 21 from the main flow path 10, the fluid A 60 can flow from the main flow path 10 into the first connection flow path 21, and furthermore the fluid A 60 can reach the end on the first flow path 11 side (dashed line 211) in the first connection flow path 21.

$P_C$ and $P_{L1}$ are each correlated with, for example, the size of the main flow path 10, the size of the first connection flow path 21, the pressure at the inlet 101 and the pressure at the outlet 102 of the main flow path 10, and the viscosity of the fluid A 60. Therefore, for example, by setting the type of the fluid A 60 introduced into the fluid handling device 1 (i.e., the viscosity of the fluid) and the liquid feed pressure of the fluid A 60 (i.e., the pressure at the inlet 101 and the pressure at the outlet 102), the structures of the main flow path 10 and the first connection flow path 21 can be designed such that Pc and $P_{L1}$ satisfy "$P_C>P_{L1}$". The designs of the flow paths can be set by using, for example, an expression described later. Note that there are no limitations on the size of the first flow path 11.

The following describes examples of the relationship between Pc and $P_{L1}$, the size of the main flow path 10, the size of the first connection flow path 21, the pressure at the inlet 101 and the pressure at the outlet 102 of the main flow path 10, and the viscosity of the fluid A 60.

Pc in Condition 1, that is, the internal pressure Pc of the branching portion 50 of the main flow path 10, for example, can be expressed by the following expressions. According to Pc in the following expressions, the fluid A 60 introduced into the main flow path 10 flows downstream and gradually flows into the first connection flow path 21 in the branching portion 50.

[Math 1]

$$R_{Out} = \frac{2\mu \cdot l_{Out}(2w_{Out} + 2d_{Out})^2}{(w_{Out}d_{Out})^3}$$

$$R_{In} = \frac{2\mu \cdot l_{In}(2w_{In} + 2d_{In})^2}{(w_{In}d_{In})^3}$$

$$P_C - P_{Out} = Q \cdot R_{Out}$$

$$P_{In} - P_C = Q \cdot R_{In}$$

$$P_C = \frac{R_{Out}P_{In} + R_{In}P_{Out}}{R_{In} + R_{Out}}$$

$$= P_{In} \frac{l_{Out}(2w_{Out} + 2d_{Out})^2(w_{In}d_{In})^3}{l_{In}(2w_{In} + 2d_{In})^2(w_{Out}d_{Out})^3 + l_{Out}(2w_{Out} + 2d_{Out})^2(w_{In}d_{In})^3}$$

The symbols in the above expressions are as shown in FIG. 3 and as follows.

$R_{Out}$: flow path resistance of outlet flow path 104 in main flow path 10 (unit: kPa·s/mm³)
$R_{In}$: flow path resistance of inlet flow path 103 in main flow path 10 (unit: kPa·s/mm³)
Q: flow rate in main flow path 10 in branching portion 50 (unit: mm³/s)

When the fluid A 60 is fed with positive pressure from the inlet 101 of the main flow path 10, the pressure at the inlet 101 is the liquid feed pressure $P_{In}$, and the pressure at the outlet 102 is atmospheric pressure (i.e., 0 kPa), and thus the internal pressure $P_C$ in the branching portion 50 in the main flow path 10 is expressed by the following expression. Note that

**EP 4 474 468 A1**

there is no limitation to this, and the fluid A 60 may be fed with negative pressure from the inlet 101 of the main flow path 10 (e.g., suctioned from the outlet 102), and in this case, the above description similarly applies with the exception that the pressure $P_{In}$ at the inlet 101 is atmospheric pressure, and the pressure at the outlet 102 is the suction pressure $P_{out}$.

[Math 2]

$$P_C = P_{In} \frac{R_{Out}}{R_{In} + R_{Out}}$$

Next, $P_{L1}$ in Condition 1, that is, the Laplace pressure at which the dynamic contact angle θ is less than π (rad), can be expressed by, for example, the following expression. $P_{L1}$ is a function expressed by the cross-sectional shape of the first connection flow path 21, the material forming the inner wall of the first connection flow path 21, and the interfacial tension. The interfacial tension may be, for example, the tension acting at the interface between a gas and a liquid material, or the tension acting on the interface between one liquid material and a different liquid material. In the present embodiment, the interface is, for example, the interface between the fluid A 60 introduced from the main flow path 10 into the first connection flow path 21 and the fluid B 80 (see FIG. 4) filling the first connection flow path 21 in advance. As described above, for example, a combination is possible in which the fluid A 60 is a liquid material that has fluidity and the fluid B 80 is a gas or a liquid material different from the fluid A 60. The symbols in the following expression are as shown in FIG. 3.

[Math 3]

$$P_{L1} = f\left(w_{Slit}, d_{Slit}, \text{ material forming inner wall of connection flow path, fluid A, fluid B}\right)$$

The expression of $P_{L1}$ is, for example, more specifically represented by the following expression. θ in the following expression, that is, the dynamic contact angle θ of the fluid A 60, is a factor determined by the material forming the inner wall of the first connection flow path 21 and the types of the fluid A 60 and the fluid B 80. The other symbols in the above expression are as shown in FIG. 3 and as follows. The interfacial tension γ is a constant determined by, for example, the type of the fluid A 60 (e.g., fluid colloid) and the type of the fluid B 80 (e.g., air) filling the first connection flow path 21 in advance. The dynamic contact angle θ is a constant determined by the fluid A 60, the fluid B 80, and the material forming the inside of the first connection flow path 21.

γ: interfacial tension at interface between fluid A 60 and fluid B 80 (mN/mm)
θ: dynamic contact angle (rad)

[Math 4]

$$P_{L1} = \gamma\left(\frac{-2\cos\theta}{w_{Slit}} + \frac{-2\cos\theta}{d_{Slit}}\right)$$

As described above, in the fluid handling device 1 of the present embodiment, due to satisfying Condition 1, the fluid A 60 flowing into the first connection flow path 21 can flow to the end on the first flow path 11 side (dashed line 211) in the first connection flow path 21. In this case, if there is a limit on the time required for the fluid A 60 to flow from the end on the main flow path 10 side to the end on the first flow path 11 side (dashed line 211) in the first connection flow path 21, for example, it is preferable that Condition 3 of the following expression is further satisfied.

[Math 5]

$$\int_0^{T_{Lim}} Q_{Slit}(t)dt \geq w_{Slit}d_{Slit}l_{Slit}$$

[Math 6]

Here, $Q_{Slit}(t)$ satisfies the following:

$$P_C - (P_H + P_{L1}) = Q_{Slit}(t)R_{Slit}(t)$$

$$R_{Slit}(t) = \frac{2\mu \cdot G(t)(2w_{Slit} + 2d_{Slit})^2}{(w_{Slit}d_{Slit})^3}$$

$$G(t) = \frac{1}{w_{Slit}d_{Slit}} \int_0^t Q_{Slit}(t)dt$$

$$P_C - P_{L1} = \frac{2\mu(2w_{Slit}+2d_{Slit})^2}{(w_{Slit}d_{Slit})^4} Q_{Slit}(t) \int_0^t Q_{Slit}(t)dt$$

The symbols in the above expressions are as shown in FIGS. 3 and 4 and as follows.

$Q_{Slit}(t)$: flow rate of fluid A introduced from main flow path 10 into the first connection flow path 21 per unit time, at specific time (t) (unit: $mm^3$/s)
$\int Q_{Slit}(t)dt$: total amount of fluid introduced from main flow path 10 into first connection flow path 21 up to specific time (t)
$T_{Lim}$: time limit (unit: s)
$G(t)$: distance that fluid A 60 moved in the first connection flow path 21 (mm)

The above expressions (Math 6), for example, means the following. In the above expression, "$P_C$-($P_H$+$P_{L1}$)" is the pressure required to push the fluid A 60 to the front end of the fluid A 60 flowing from the branching portion 50 of the main flow path 10 to the first connection flow path 21, and the internal pressure $P_H$ of the first flow path 11 is normally atmospheric pressure (zero). In the case where the cross-sectional shape of the first connection flow path 21 in the flow direction is constant, that is, in the case where the Laplace pressure $P_{L1}$, the flow path width wsiit, and the flow path depth $d_{Slit}$ are constant, then $Q_{Slit}(t)\int Q_{Slit}(t)dt$ is a constant, and Qsiit(t) and $\int Q_{Slit}(t)dt$ are in an inversely proportional relationship. Therefore, as the total amount (flow rate integral $\int Q_{Slit}(t)dt$) of the fluid A 60 introduced from the branching portion 50 into the first connection flow path 21 up to the specific time (t) increases, the flow rate $Q_{Slit}(t)$ per unit time at the specific time (t) decreases. Here, if the flow rate Qsiit(t) per unit time at the specific time (t) is small, this means that the fluid A 60 flows less easily, and the flow path resistance $R_{Slit}(t)$ of the first connection flow path 21 is large.
Although the movement of the fluid A 60 to the first connection flow path 21 that connects the main flow path 10 to the first flow path 11 has been described as an example in the present embodiment, the present invention is not limited to this. In the case where the fluid handling device 1 further includes the second flow path 12 and the second connection flow path 22, for example, the fluid A 60 introduced into the main flow path 10 can be similarly introduced into the second connection flow path 22 by replacing the first flow path 11 with the second flow path 12 and replacing the first connection flow path 21 with the second connection flow path 22 in the above description. This similarly applies to other embodiments as well.

(Second Embodiment)

According to the first embodiment, if Condition 1 is satisfied, the fluid A 60 can flow in the first connection flow path 21 up to the end on the first flow path 11 side (dashed line 211) in the first connection flow path 21. At this time, it is desirable that the fluid A 60 stops at the end on the first flow path 11 side (dashed line 211) in the first connection flow path 21 and does not flow into the first flow path 11. Therefore, in the fluid handling device of the present embodiment, it is preferable that the main flow path 10, the first flow path 11, and the first connection flow path 21 further satisfy Condition 2 in addition to Condition 1 in a state where the fluid A 60 is introduced into the main flow path 10.
$$\text{Condition 2: } P_C < P_{L2},$$

,

$P_C$: an internal pressure in branching portion of main flow path branching to connection flow path, and

$P_{L2}$: a Laplace pressure when dynamic contact angle $\theta$ of fluid A introduced from main flow path into connection flow path relative to inner wall of connection flow path is set to $\pi$.

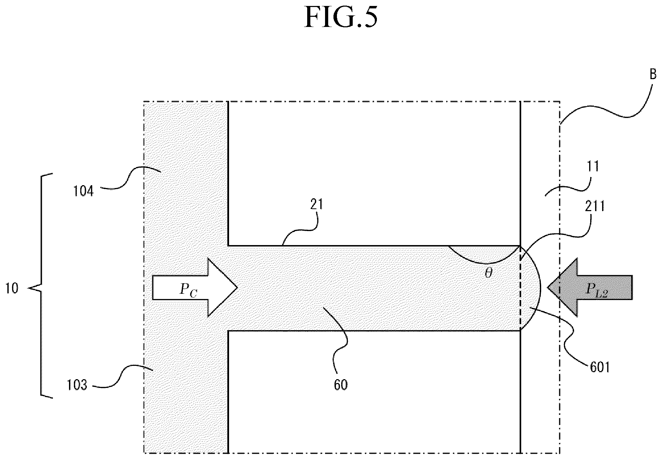

Condition 2 will be described below with reference to the schematic diagrams of FIGS. 3 and 5. FIG. 5 is an enlarged view of a dashed-dotted line region B in FIG. 2, and shows a state when the fluid A 60 is introduced into the main flow path 10.

The following specific example is a description of the main flow path 10, the first flow path 11, and the first connection flow path 21 of the fluid handling device 1, but the description similarly applies to the second flow path 12 and the second connection flow path 22 in FIG. 1. That is, the description of the main flow path 10, the first flow path 11, the first connection flow path 21, and the relationship therebetween in the present embodiment can be applied to the main flow path 10, the second flow path 12, the second connection flow path 22, and the relationship therebetween. Therefore, in Condition 2 regarding the main flow path 10 and the first connection flow path 21, the "connection flow path" in $P_C$ and $P_{L1}$ is the first connection flow path 21 that connects the main flow path 10 to the first flow path 11, and in Condition 2 regarding the main flow path 10 and the second connection flow path 22, the "connection flow path" in $P_C$ and $P_{L1}$ is the second connection flow path 22 that connects the main flow path 10 to the second flow path 12.

$P_C$ in Condition 2 is the same as $P_C$ in Condition 1 in the first embodiment. The "dynamic contact angle" in $P_{L2}$ in Condition 2 is, for example, the contact angle (advancing contact angle) $\theta$ of the surface 601 of the fluid A 60 relative to the inner wall of the first connection flow path 21 in FIG. 5.

As shown in FIG. 5, Pc is the internal pressure in the branching portion 50 of the main flow path 10 that branches to the first connection flow path 21, and $P_{L2}$ is the Laplace pressure (unit: kPa) when the dynamic contact angle $\theta$ of the fluid A 60 introduced from the main flow path 10 into the first connection flow path 21 relative to the inner wall of the first connection flow path 21 is set to $\pi$ (rad) (i.e., when $\theta$=180°). When dynamic contact angle $\theta$=$\pi$ (rad), this means that the advancing free surface 601 of the fluid A 60 has moved beyond the end on the first flow path 11 side (dashed line 211) in the first connection flow path 21. In the fluid handling device 1, due to the Laplace pressure $P_{L2}$ satisfying Condition 2 "$P_C<P_{L2}$" when the fluid A 60 introduced into the main flow path 10 is introduced into the first connection flow path 21 from the main flow path 10, the fluid A 60 can flow from the main flow path 10 into the first connection flow path 21 and reach the end on the first flow path 11 side (dashed line 211) in the first connection flow path 21, and furthermore, the flow can be stopped at that end.

$P_C$ and $P_{L2}$ are each correlated with, for example, the size of the main flow path 10, the size of the first connection flow path 21, the pressure at the inlet 101 and the pressure at the outlet 102 of the main flow path 10, and the viscosity of the fluid A. Therefore, assuming that the type of the fluid A to be introduced into the fluid handling device 1 (i.e., the viscosity of the fluid) and the liquid feed pressure of the fluid A 60 (i.e., the pressure at the inlet 101 and the pressure at the outlet 102) are known, the structures of the main flow path 10 and the first connection flow path 21 can be set such that Pc and $P_{L2}$ satisfy "$P_C<P_{L2}$". The designs of the flow paths can be set by using, for example, an expression described later.

The following describes examples of the relationship between $P_C$ and $P_{L2}$, the size of the main flow path 10, the size of the first connection flow path 21, the pressure at the inlet 101 and the pressure at the outlet 102 of the main flow path 10, and the viscosity of the fluid A 60. Aspects not specifically described are similar to the description of $P_C$ and $P_{L1}$ in the first embodiment.

$P_{L2}$ in Condition 2, that is, the Laplace pressure at which the dynamic contact angle $\theta$ is $\pi$ (rad), can be expressed by the same expression as $P_{L1}$ in Math 3 in the first embodiment, for example, and more specifically, is expressed by the following expression. $\theta$ in the following expression, that is, the dynamic contact angle $\theta$ of the fluid A 60, is a factor determined by the material forming the inner wall of the first connection flow path 21 and the type of the fluid. The other symbols in the above expression are as shown in FIG. 3 and as follows.

$\gamma$: interfacial tension at interface between fluid A 60 and fluid B 80 (mN/mm)

$\theta$: dynamic contact angle (rad)

[Math 7]

$$P_{L2} = \gamma \left( \frac{-2\cos\theta}{w_{Slit}} + \frac{-2\cos\theta}{d_{Slit}} \right)$$

In the state shown in FIG. 5, the dynamic contact angle $\theta$ is the maximum value $\pi$, and therefore, $P_{L2}$ is expressed as follows.

[Math 8]

$$P_{L2}Max = \gamma\left(\frac{2}{w_{Slit}} + \frac{2}{d_{Slit}}\right)$$

(Third Embodiment)

An example of designing the fluid handling device 1 based on Condition 1 of the first embodiment and Condition 2 of the second embodiment will be described below.

Here, it is assumed that the fluid A 60 introduced into the main flow path 10 is water, a fluid B 80 filling the first connection flow path 21 in advance is air, the interfacial tension (surface tension) between the fluid A 60 and the fluid B 80 is 72.8 mN/m, the dynamic contact angle θ is 110°, the cross-sectional shape of the first connection flow path 21 is square, and the width and the depth of the first connection flow path 21 are each 0.05 mm. In this case, $P_{L1}$ is about 1.16 kPa based on Math 4 above, and $P_{L2}$ is about 5.82 kPa based on Math 8 above. Therefore, in the fluid handling device 1, $P_C$ in Condition 1 and Condition 2 ($P_{L1}<P_C<P_{L2}$) preferably is in the relationship of about 2 kPa < Pc < 5 kPa.

There are no particular limitations on the size of the fluid handling device 1 that satisfies the above condition. The upper limits of the width and the depth of the first connection flow paths 21 are, for example, 0.3 mm or less and 0.1 mm or less, respectively, from the viewpoint of maintaining the Laplace pressure at a certain level, and, for example, can be about 0.05 mm from the viewpoint of practical use as the blood vessel model as described above. The upper limits of the width and the depth of the main flow path 10 (e.g., in the vicinity of the branching portion 50) are, for example, 1 mm or less, 0.5 mm or less, or 0.3 mm or less.

(Fourth Embodiment)

In the fluid handling device 1, the first connection flow path 21 may have a constant cross-sectional area along the direction of travel of the fluid A 60, as illustrated in FIG. 3, or may have a shape in which the cross-sectional area on the first flow path 11 side is smaller than the cross-sectional area on the main flow path 10 side. In the latter case, for example, the flow path depth of the first connection flow path may be constant and the flow path width of the first connection flow path may decrease from the main flow path side toward the first flow path side, or the flow path width of the first connection flow path may be constant and the flow path depth of the first connection flow path may decrease from the main flow path side toward the first flow path side, or both the flow path width and the flow path depth of the first connection flow path may decrease from the main flow path side toward the first flow path side. The cross section of the flow path is, for example, the cross section of the hollow portion of the flow path, and the cross-sectional area of the flow path is, for example, the cross-sectional area of the hollow portion (this similarly applies hereinafter).

The cross-sectional shape of the first connection flow path 21 is not particularly limited, and may be, for example, polygonal or circular. The polygon is, for example, a quadrangle. Specific examples of the quadrangle include a rectangle (e.g., a square, a rectangle, or the like), a trapezoid, and the like. In the case where the cross-sectional shape is circular, for example, a shape in which the diameter decreases from the main flow path side toward the first flow path side is preferable.

If the cross-sectional area of the first connection flow path 21 is smaller on the first flow path 11 side than on the main flow path 10 side as in the present embodiment, the Laplace pressure $P_{L2}$ at θ=π can be increased.

(4-1)

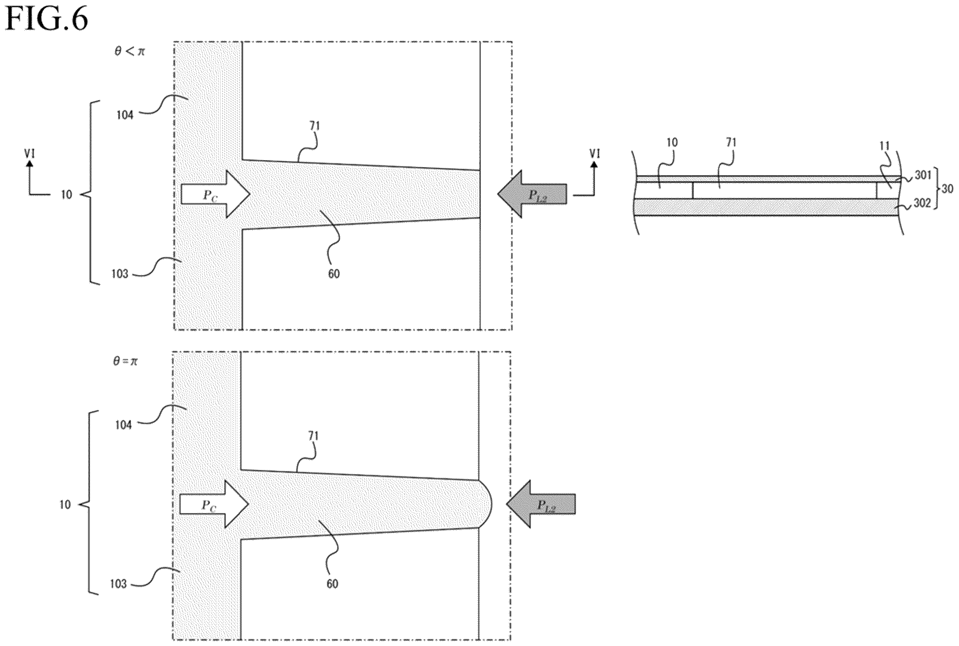

FIG. 6 shows an example of a first connection flow path 71 in the present embodiment. In FIG. 6, the left drawing is an enlarged view of the dashed-dotted line region B in FIG. 2, as in FIG. 4, and the right drawing is a cross-sectional view taken along line VI-VI in the enlarged view. The first connection flow path 71 of FIG. 6 has, for example, a rectangular (e.g., square or oblong) or trapezoidal cross-sectional shape. In FIG. 6, the first connection flow path 71 has a shape in which the cross-sectional area is smaller at the end portion on the first flow path 11 side than at the end portion on the main flow path 10 side, and specifically, for example, has a shape in which the cross-sectional area decreases in a stepwise or continuous manner in the flow direction of the first connection flow path 71 (from the end portion on the main flow path 10 side toward the end portion on the first flow path 11 side). As shown in the right drawing of FIG. 6, the first connection flow path 71 has a constant depth in the flow direction of the fluid A 60, and as shown in the left drawing of FIG. 6, has a width that decreases from the main flow path 10 toward the first flow path 11. In the left drawing of FIG. 6, the upper drawing shows a state where the dynamic contact angle θ of the fluid A 60 is less than π (θ<π), and the lower drawing shows a state where the dynamic contact angle θ of the fluid A 60 is π (θ=π).

(4-2)

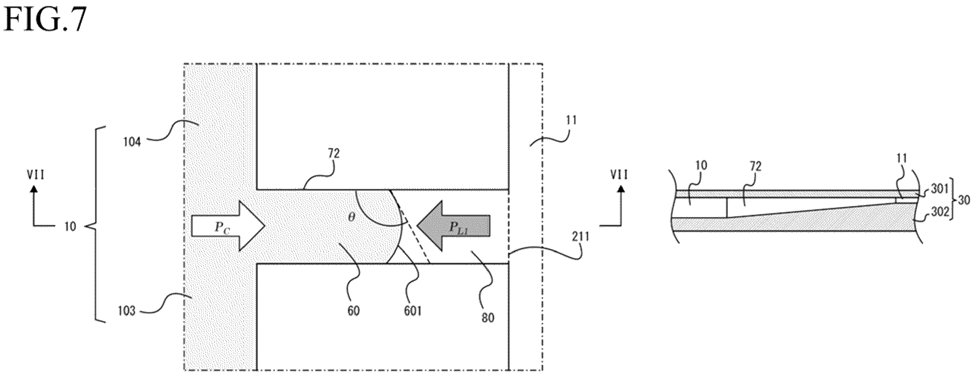

FIG. 7 shows an example of a first connection flow path 72 in the present embodiment. In FIG. 7, the left drawing is an enlarged view of the dashed-dotted line region B in FIG. 2, as in FIG. 4, and the right drawing is a cross-sectional view taken along line VII-VII in the enlarged view. The first connection flow path 72 of FIG. 7 has, for example, a rectangular (e.g., square or oblong) or trapezoidal cross-sectional shape. Similarly to (4-1) above, the first connection flow path 72 of FIG. 7 also has a cross-sectional area that changes, but as shown in the left drawing of FIG. 7, the width of the first connection flow path 72 is constant, and as shown in the right drawing of FIG. 7, the depth in the flow direction of the fluid A 60 becomes shallower from the main flow path 10 side toward the first flow path 11. The depth of the first flow path 11, for example, may be the same as the depth of the opening portion (the opening at the end on the first flow path 11 side) of the first connection flow path 72, may be shallower than the depth of the opening portion of the first connection flow path 72, or may be the same as the depth of the main flow path 10.

(4-3)

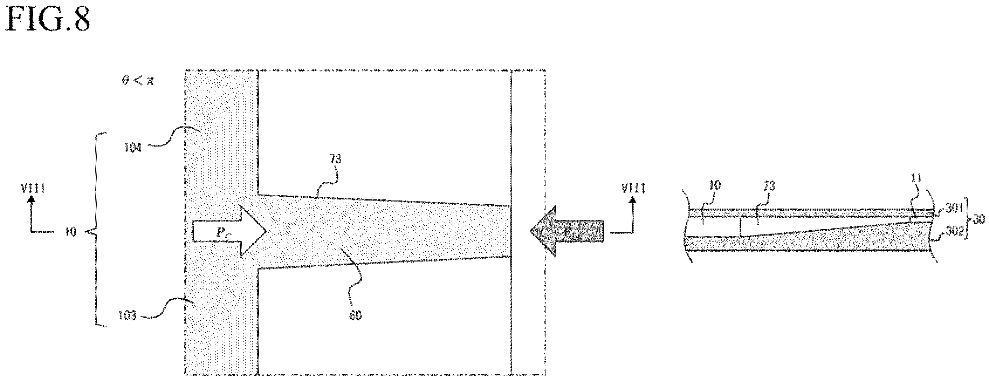

FIG. 8 shows an example of a first connection flow path 73 in the present embodiment. In FIG. 8, the left drawing is an enlarged view of the dashed-dotted line region B in FIG. 2, as in FIG. 4, and the right drawing is a cross-sectional view of the first connection flow path 73 taken along line VIII-VIII. The first connection flow path 73 of FIG. 8 has, for example, a rectangular (e.g., square or oblong) or trapezoidal cross-sectional shape. Similarly to (4-1) and (4-2) above, the first connection flow path 73 of FIG. 8 also has a cross-sectional area that changes, but as shown in the left drawing of FIG. 8, the width of the first connection flow path 73 decreases from the main flow path 10 toward the first flow path 11, and as shown in the right drawing of FIG. 8, the depth in the flow direction of the fluid A 60 becomes shallower from the main flow path 10 side toward the first flow path 11. The depth of the first flow path 11, for example, may be the same as the depth of the opening portion (the opening at the end on the first flow path 11 side) of the first connection flow path 73, may be shallower than the depth of the opening portion of the first connection flow path 73, or may be the same as the depth of the main flow path 10.

(Fifth Embodiment)

As an example of use of the fluid handling device 1, for example, as described above, there is a method of introducing the fluid A 60 (e.g., a sol) into the main flow path 10 and the first connection flow path 21, and then introducing a fluid C (e.g., a culture medium) containing HUVEC cells into the first flow path 11. At this time, it is desirable that the fluid A 60 introduced into the first connection flow path 21 is not pushed back to the main flow path 10 by the introduction of the fluid C into the first flow path 11.

Therefore, in the fluid handling device of the present embodiment, it is preferable that, in the first flow path 11, the flow path resistance from the inlet 111 to the portion in communication with the first connection flow path 21 is relatively large, and the flow path resistance from the portion in communication with the first connection flow path 21 to the outlet 112 is relatively small. According to this configuration, the pressure acting in the first connection flow path 21 decreases, and, for example, the fluid A 60 introduced into the first connection flow path 21 is less likely to be pushed back toward the main flow path 10.

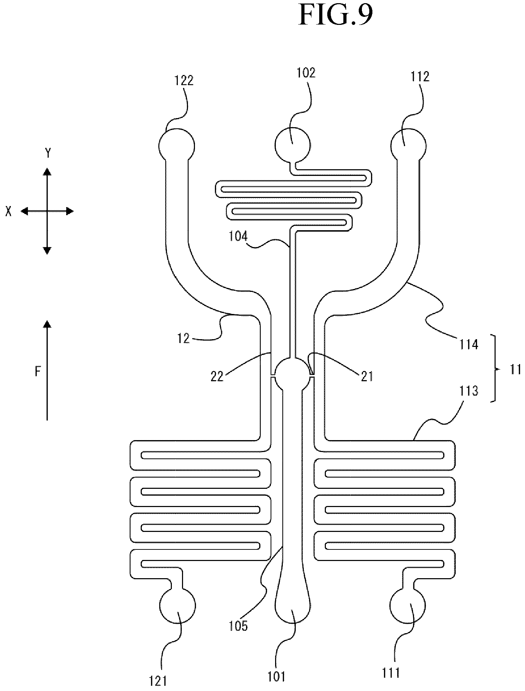

FIG. 9 shows an example of the fluid handling device according to the present embodiment. FIG. 9 is a plan view schematically showing the shapes of flow paths in the fluid handling device of the present embodiment. The first flow path 11 in the fluid handling device has a structure in which the length of the inlet flow path 113 on the inlet 111 side is relatively long and the length of the outlet flow path 114 on the outlet 112 side is relatively short. With such a structure, the flow resistance from the branching portion 50, which branches to the first connection flow path 21, to the outlet 112 can be made smaller than the flow resistance from the inlet 111 to the branching portion 50.

(Sixth Embodiment)

The fluid handling device of the present invention can be set according to Condition 1 and optionally Condition 2 as described above, and is preferably designed within a range of the following scale from the viewpoint of handling and the like.

Regarding overall size, the fluid handling device 1 illustrated in FIG. 1 as an example is, for example, 10 to 100 mm wide in the X direction, 10 to 100 mm long in the Y direction, and 0.5 to 3 mm thick in the Z direction.

The cross-sectional shape of the internal space of the main flow path 10 in the direction orthogonal to the Y direction may be, for example, a polygon or a circle (e.g., a perfect circle or an ellipse). The polygon is, for example, a quadrangle. Specific examples of the quadrangle include a rectangle (e.g., a square, a rectangle, or the like), a trapezoid, and the like. In the case where the cross section is circular, the diameter thereof may be set such that the cell mass can move, for example, and specific examples of the diameter include 200 to 1000 $\mu$m, 300 to 800 $\mu$m, and 400 to 700 $\mu$m. In the case where the cross section of the main flow path 10 is quadrangular, the width and the depth thereof are, for example, 200 to 1000 $\mu$m. The length of the main flow path 10 in the flow direction F is, for example, 0.5 to 80 mm.

There are no limitations on the size and the shape of the trap portion 40 in the main flow path 10 as long as it can trap the cell mass flowing together with the fluid A 60 in the main flow path 10 while allowing the fluid A 60 to flow. The trap portion 40 may be, for example, shaped as a protrusion that protrudes in the Z direction on the inner wall of the main flow path 10.

As described above, when forming a blood vessel model using the inner wall of the first flow path 11 as a scaffold, the shape and size of the inner peripheral surface (the cross section of the internal space) of the first flow path 11 are preferably the same as or similar to the shape and size of the outer peripheral surface of the blood vessel. The cross-sectional shape of the internal space of the first flow path 11 in the direction orthogonal to the Y direction may be, for example, a polygon or a circle (e.g., a perfect circle or an ellipse). The polygon is, for example, a quadrangle. Specific examples of the quadrangle include a rectangle (e.g., a square, a rectangle, or the like), a trapezoid, and the like. In the case where the cross section is circular, the diameter is, for example, 100 to 500 $\mu$m, 150 to 400 $\mu$m, or 200 to 300 $\mu$m, and in the case where the cross section is a quadrangle, the width and the depth are, for example, 100 to 500 $\mu$m, 150 to 400 $\mu$m, or 200 to 300 $\mu$m. The length of the first flow path 11 in the flow direction F is, for example, 0.5 to 80 mm.

The number of first connection flow paths 21, for example, may be one or more. The cross-sectional shape of the internal space of the first connection flow path 21 in the direction orthogonal to the X direction may be, for example, a polygon or a circle (e.g., a perfect circle or an ellipse). The polygon is, for example, a quadrangle. Specific examples of the quadrangle include a rectangle (e.g., a square, a rectangle, or the like), a trapezoid, and the like. The size of the cross section of the first connection flow path 21 is preferably a size that allows the passage of, for example, a substance released from the cell mass in the main flow path 10, a substance or cells released from the blood vessel model in the first flow path 11, cells (e.g., immune cells) that are to pass through the blood vessel model, or sprouts or the like extending from the blood vessel model, but does not allow passage of the cell mass. In the case where the cross section is circular, the diameter is, for example, 10 to 100 pm, 20 to 90 pm, 30 to 80 pm, or 50 to 150 pm. In the case where the cross section is quadrangular, the width and the depth thereof are, for example, 10 to 100 pm, 20 to 90 pm, 30 to 80 pm, or 50 to 150 pm. The length of the first connection flow path 21 in the X direction is, for example, 10 to 500 pm, 50 to 300 pm, or 100 to 200 pm.

The position at which the first connection flow path 21 is connected to the main flow path 10 is not particularly limited, and is, for example, upstream of the trap portion 40 of the main flow path 10. In the main flow path 10, the distance between the upstream end of the trap portion 40 and the downstream end of the position of connection to the first connection flow path 21 is, for example, 0 to 1000 pm, 0 to 500 pm, or 0 to 200 pm.

In the case where the fluid handling device 1 further includes the second flow path 12 and the second connection flow path 22, the size and shape of these flow paths are not particularly limited, and for example, the description of the first flow path 11 can be applied to the second flow path 12, and the description of the first connection flow path 21 can be applied to the second connection flow path 22.

In the fluid handling device 1, the fluid A 60 introduced into the main flow path 10 is, for example, a medium that can move inside the main flow path 10 by positive pressure or negative pressure applied to the main flow path 10, and will also be referred to as a flowable medium. The fluid A 60 is, for example, a non-gaseous flowable medium, such as a liquid. The fluid A 60 corresponding to the liquid material is, for example, a liquid, a fluid colloid (sol), or the like. The liquid is an aqueous liquid. The aqueous liquid may be any of, for example, a solvent, a solution, a suspension, a mixed liquid, and the like, and specific examples thereof include water, a buffer solution, a culture medium, and the like. The fluid colloid (sol) is preferably, for example, one that becomes a non-fluid colloid (gelled) by polymerization or the like after introduction into the main flow path 10 and the first connection flow path 21. Examples of the fluid colloid include TYPE I COLLAGEN SOLUTION (trade name, manufactured by Human Corporation). In the various expressions described above, the viscosity, the dynamic contact angle, the interfacial tension (e.g., surface tension), and the like can be set based on the type of the fluid A 60.

The following describes specific examples of methods of using the fluid handling device 1.

(1) First, a sol (fluid A 60) containing a cell mass is introduced through the inlet 101 of the main flow path 10, and the sol flows to the outlet 102 side of the main flow path 10. Due to the introduction of the sol, the sol fills the main flow path 10, the first connection flow path 21, and the second connection flow path 22. When the sol is introduced into the main flow path 10, the fluid B, which has been introduced into the first connection flow path 21 and the second connection flow path 22, is not particularly limited, and is, for example, a gas such as air.

The cell mass moves in the flow direction (arrow F) together with the sol and is trapped by the trap portion 40 of the main flow path 10. After introduction of the sol, the sol undergoes conversion into a gel by polymerization or the like. The method for conversion into a gel is not particularly limited, and can be appropriately set depending on the type of the fluid colloid, and when TYPE I COLLAGEN SOLUTION (trade name, manufactured by Human Corporation) is used, for example, the fluid colloid can undergo conversion into a gel by incubation at 35 to 40°C (e.g., 37°C).

The cell mass to be introduced into the main flow path 10 is not particularly limited, and is, for example, a mass of cancer cells. The size of the cell mass is, for example, 100 to 1000 pm, 200 to 800 pm, or 300 to 500 pm.

(2) Next, a coating agent having affinity for vascular endothelial cells is introduced through the inlet 111 of the first flow path 11 and flows to the outlet 112 side of the first flow path 11. Due to filling the first flow path 11 with the coating agent, the inner wall of the first flow path is coated with the coating agent.

Examples of the coating agent include a collagen such as Collagen I. The coating liquid contains, for example, the coating agent and a solvent, and examples of the solvent include a buffer solution and a buffered saline solution such as PBS. The amount of the coating agent used, the treatment conditions such as temperature and time, and the like are not particularly limited, and for example, incubation may be performed at 25°C for 1 hour using 100 pL of the coating liquid.

(3) Then, a culture medium (fluid C) containing vascular endothelial cells for forming a blood vessel model is introduced through the inlet 111 of the first flow path 11 and flows to the outlet 112 side of the first flow path 11. Due to filling the first flow path 11 with the culture medium and culturing the cells in the first flow path 11, a tubular blood vessel model (also referred to as a pseudo blood vessel) can be formed inside the first flow path 11.

The type of the vascular endothelial cell is not particularly limited, and examples thereof include umbilical vein endothelial cells such as HUVEC cells. Also, in the formation of the blood vessel model, for example, blood vessel pericytes (pericytes) for constructing a blood vessel may be used in combination with the vascular endothelial cells. The fluid to be introduced into the first flow path 11 is not particularly limited, and can be appropriately set according to the type of the cell. The fluid is, for example, a liquid such as aqueous liquid, and is preferably a culture medium, specific examples thereof including EBM2 culture medium. The temperature condition is not particularly limited, and is, for example, room temperature during introduction and 35 to 40°C (e.g., 37°C) during culture.

(4) Next, a fluid (fluid C) containing a component involved in the growth of the cell mass is introduced through the inlet 121 of the second flow path 12 and flows to the outlet 122 side of the second flow path 12. When the second flow path 12 is filled with the component, the component moves from the second flow path 12 to the main flow path 10 via the second connection flow path 22, thus resulting in a concentration gradient of the component from the second flow path 12 toward the main flow path 10.

The component is not particularly limited, and can be appropriately set according to, for example, the type and purpose of the cell mass. Examples of the component include nutrients and growth factors such as AG (angiogenesis) factor. The temperature condition at the time of introduction is not particularly limited, and is, for example, 35 to 40°C (e.g., 37°C).

(5) The cell mass is grown in the presence of the component that has flowed from the second flow path 12. In this growth process, it is possible to reproduce, in the fluid handling device 1, a reaction in which the blood vessel model in the first flow path 11 detects the released substance from the cell mass, and a capillary vessel (also referred to as a sprout or bud of a blood vessel branch) is grown from the blood vessel model and extends to the main flow path 10 via the first connection flow path 21. Therefore, the influence of a drug can be analyzed by introducing the drug through the inlet 111 of the first flow path 11 in which the blood vessel model is formed and confirming the formation or the extension of a capillary vessel.

(Seventh Embodiment)

In the fluid handling device, a single first connection flow path 21 and a single second connection flow path 22 may be provided as shown in FIGS. 1 and 9, for example, or a plurality of each may be provided.

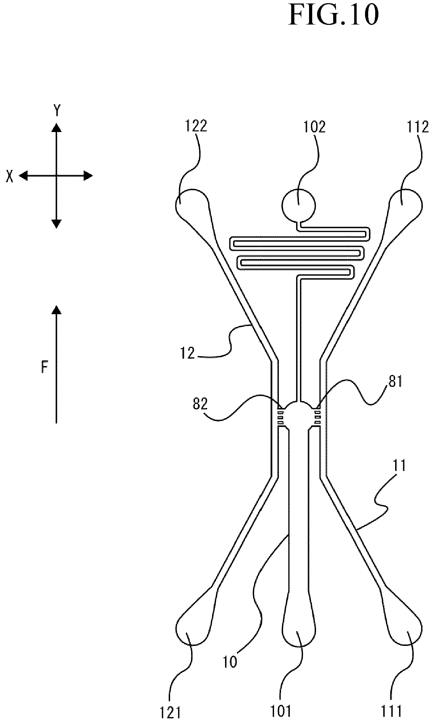

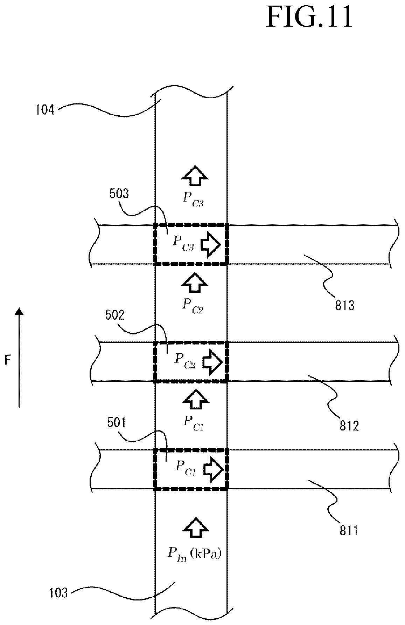

Another example of the fluid handling device of the present embodiment is shown in FIGS. 10 and 11. FIG. 10 is a plan view schematically showing the shapes of flow paths in the fluid handling device of the present embodiment. In the fluid handling device, the main flow path 10 and the first flow path 11 are in communication with each other by a first connection flow path group 81 made up of a plurality of first connection flow paths, and the main flow path 10 and the second flow path 12 are in communication with each other by a second connection flow path group 82 made up of a plurality of second connection flow paths. In the first connection flow path group 81, a plurality of first connection flow paths extending parallel to the X direction are arranged at intervals in the Y direction, and in the second connection flow path group 82. Similarly, a plurality of second connection flow paths parallel to the X direction are arranged at intervals in the Y direction. FIG. 11 is a partial view of the fluid handling device, showing a region including the first connection flow paths (811, 812, 813) in the first connection flow path group 81 and branching portions (501, 502, 503) of the main flow path 10. Although FIG. 11 illustrates a configuration in which three first connection flow paths are provided, the present invention is not limited to this.

The number of first connection flow paths that make up the first connection flow path group 81 is not particularly limited, and is, for example, 2 to 500, 2 to 300, or 2 to 100. The length of the first connection flow path group 81 in the Y direction is not particularly limited, and the distance from the upstream end portion of the first connection flow path to the downstream end portion of the first connection flow path in the X direction is, for example, 30 to 10000 pm, 30 to 5000 pm, or 30 to 1000 pm. The pitch between the first connection flow paths, that is, the distance between adjacent first connection flow paths is, for example, 5 to 100 pm, 10 to 50 pm, or 15 to 30 pm.

In this manner, in the case where the main flow path 10 is in communication with the first flow path 11 by a plurality of first connection flow paths, for example, it is preferable that the above-described Condition 1 and optionally Condition 2 are satisfied in each of the first connection flow paths and the branching portions in the main flow path 10 that correspond to the first connection flow paths. When used in a blood vessel model as described above, for example, the fluid handling device of the present invention has a compact structure, and the interval between the first connection flow paths is also small. Therefore, in the case where the fluid handling device has a plurality of first connection flow paths, the flow path resistance with the branching portions is smaller than the flow path resistance of the inlet flow path 103 and the flow path resistance of the outlet flow path 104, and therefore, for example, the partial pressures ($P_{C1}$, $P_{C2}$, $P_{C3}$) of the branching portions (e.g., 501, 502, 503) relative to the first connection flow paths are substantially the same as the internal pressure Pc.

The description of the first connection flow path group 81 and the first connection flow paths can be, for example, applied to the second connection flow path group 82 and the second connection flow paths included therein.

(Eighth Embodiment)

Regarding a method for designing and a method for manufacturing the fluid handling device of the present invention, for example, the descriptions of the first to seventh embodiments can be referred to. That is to say, in the case of designing and manufacturing a fluid handling device having the main flow path, the first flow path, and the first connection flow path described above, it is sufficient that the structures of the flow paths are designed so as to satisfy Condition 1 and optionally Condition 2. Also in the case of designing and manufacturing a fluid handling device having the second flow path and the second connection flow path, it is sufficient that the structures of the flow paths are designed so as to satisfy Condition 1 and optionally Condition 2. Thus, by providing flow path structures satisfying Condition 1 and optionally Condition 2, it is possible to design and manufacture a fluid handling device having the above-described effects to which the present invention is directed.

(Ninth Embodiment)

The fluid handling device of the present invention can be used in a method of introducing a fluid into a fluid handling device of the present invention, for example, but the present invention is not limited to this. In other words, according to the introduction method of the present invention, for example, effects to which the present invention is directed can be achieved by introducing the fluid into an existing fluid handling device in such a manner that Condition 1 is satisfied and optionally Condition 2 is satisfied.

In the introduction method of the present embodiment, if the fluid is introduced into the main flow path such that the pressure relationship of Condition 1 is satisfied, the main flow path and the first connection flow path can be filled with the fluid. Regarding the pressure relationship of Condition 1, the pressure at the inlet 101 and the pressure at the outlet 102 of the main flow path 10 can be set based on the condition of introducing the fluid based on the sizes of the main flow path and the first connection flow path in the fluid handling device to be used, the material forming the inner wall of the first connection flow path, the interfacial tension (e.g., surface tension) of the fluid to be used, the type of the fluid B (e.g., gas) filling the first connection flow path in advance, and the expressions.

(Tenth Embodiment)

As described above, with the fluid handling device, a blood vessel model can be formed in the first flow path by introducing a liquid containing cells into the first flow path and culturing the cells, and blood flow in a human body can be reproduced by circulating a liquid such as blood or pseudo-blood and a liquid containing a drug or the like in the first flow path in which the blood vessel model is formed. As described above, by circulating the liquid through the first flow path, the fluid handling device of the present embodiment can be used as a pseudo model of a blood vessel and blood flow in a human body.

Therefore, an example of a fluid handling device particularly preferable for circulating a liquid as a fluid in the first flow path will be described in the present embodiment. Note that the present embodiment can also be used in a case where the liquid is not circulated. The description of the embodiments can be applied to the fluid handling device of the embodiment unless otherwise specified.

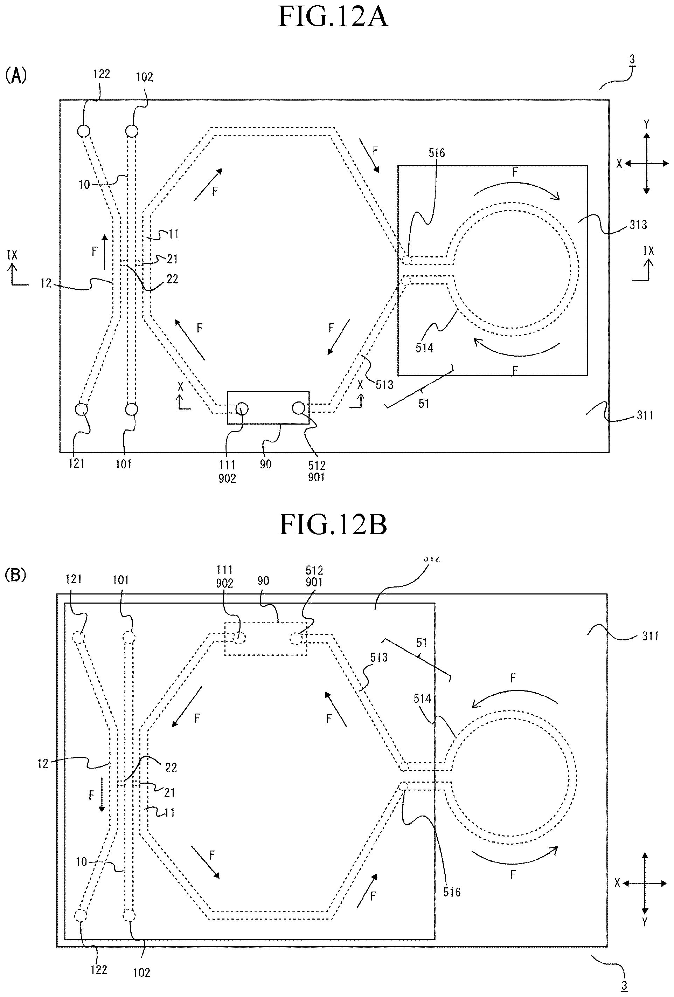

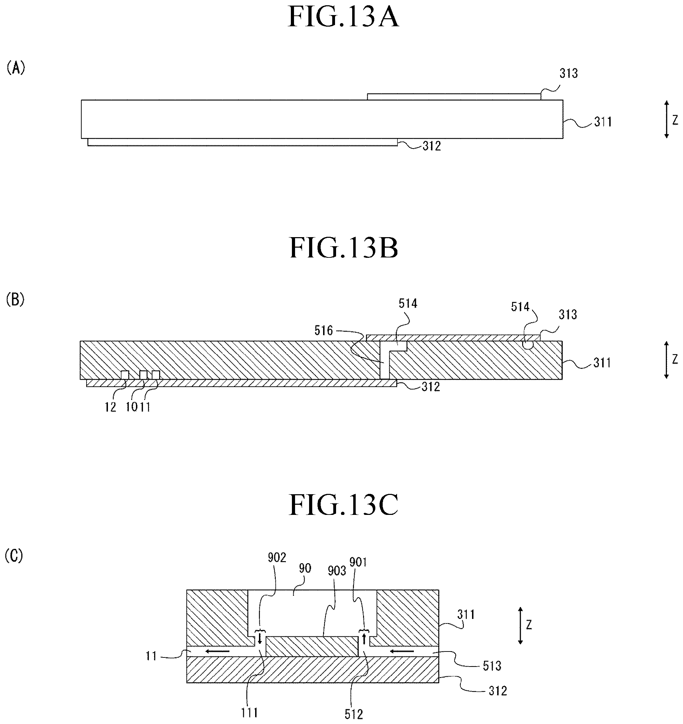

FIGS. 12 and 13 show an example of the fluid handling device of the present embodiment further including a circulation flow path and a reservoir for storing a fluid. In FIG. 12, (A) is a plan view (top view) of the fluid handling device as viewed from the upper surface side, and (B) is a plan view (bottom view) of the fluid handling device as viewed from the lower surface side. In FIG. 13, (A) is a side view of the fluid handling device, (B) is a cross-sectional view taken along line IX-IX in FIG. 12(A), and (C) is a cross-sectional view of a region including the reservoir as viewed from the X-X direction in the fluid handling device in FIG. 12(A). In the figure, an arrow F indicates the flow direction of the liquid.

The fluid handling device 3 includes a substrate 311, a cover 312, and a cover 313. One surface (the upper surface in FIG. 13, hereinafter called the "upper surface") of the substrate 311 has a recessed portion that forms a contact region 514 of a circulation flow path 51, which is an internal flow path, and a recessed portion that forms a reservoir 90. Also, the substrate 311 further has through holes that penetrate to the other surface (the lower surface in FIG. 13, hereinafter called the "lower surface") at two ends 516 of the contact region 514 and at two locations 901 and 902 in the bottom portion of the recessed portion that forms the reservoir 90. On the other hand, a recessed portion that forms the main flow path 10, the first flow path 11, the second flow path 12, the first connection flow path 21, the second connection flow path 22, and an outlet-side region 513 of the circulation flow path 51, which are internal flow paths, is formed in the lower surface of the substrate 311. In the substrate 311, the through holes 516 corresponding to two ends of the contact region 514 of the circulation flow path 51 are respectively in communication with the outlet-side region 513 of the circulation flow path 51 and the recessed portion of the first flow path 11, and the two through holes 901 and 902 at the bottom portion of the reservoir 90 are respectively in communication with the recessed portion of the outlet-side region 513 of the circulation flow path 51 and the recessed portion of the first flow path 11.

Also, the cover 313 is disposed on the upper surface of the substrate 311 so as to expose the recessed portion that forms the reservoir 90 and cover the recessed portion that forms the internal flow path, and the cover 312 is disposed on the lower surface of the substrate 311 so as to cover the recessed portion that forms the internal flow path. By adopting such a structure, the upper surface side of the substrate 311 has formed therein the contact region 514 of the circulation flow path 51 and the reservoir 90 that is open upward, and the lower surface side of the substrate 311 has formed therein the main flow path 10, the first flow path 11, the second flow path 12, the first connection flow path 21, the second connection flow path 22, and the outlet-side region 513 of the circulation flow path 51. The contact region 514 of the circulation flow path 51 on the upper surface side of the substrate 311 is connected, in the vertical direction, to the first flow path 11 and the outlet-side region 513 of the circulation flow path 51 on the lower surface side of the substrate 311 by the through hole 516, and the reservoir 90 on the upper surface side of the substrate 311 is connected, in the vertical direction, to the first flow path 11 and the outlet-side region 513 of the circulation flow path 51 on the lower surface side of the substrate 311 by the through holes 901 and 902.

Note that the upper surface of the substrate 311 may further have, for example, through holes forming inlets 101 and 121 and outlets 102 and 122 at locations corresponding to the two ends of the main flow path 10 and the two ends of the second flow path 12.

The substrate 311 is, for example, a molded body whose shape does not change when the fluid handling device 3 is used, and is preferably a hard substrate made of, for example, a resin. The type of the resin is not particularly limited. The cover 312 that covers recessed portions such as the main flow path 10 on the lower surface of the substrate 311 is, for example, a resin cover; may be hard, soft, or elastic; is not limited in terms of thickness; and may be, for example, a film or a sheet. The cover 313 that covers the recessed portion of the contact region 514 of the circulation flow path 51 is, for example, a resin cover; may be hard, soft, or elastic; is not limited in terms of thickness; and may be, for example, a film or a sheet.

In the fluid handling device 3, the reservoir 90, the first flow path 11, and the circulation flow path 51 are also referred to as a circulation route of the first flow path 11.

The reservoir 90 is, for example, a tank capable of storing a fluid; is open at the top; and has two through holes 901 and 902. The through holes 901 and 902 are, for example, preferably formed in a bottom portion 903 of the tank. Due to connecting the through hole 902 and the inlet 111 of the first flow path 11, the reservoir 90 is in communication with the first flow path 11, and due to connecting the through hole 901 and the first end 512 of the circulation flow path 51 (the outlet-side region 513), the reservoir 90 is in communication with the circulation flow path 51.

The circulation flow path 51 includes the outlet-side region 513 in communication with the reservoir 90 and the contact region 514 in contact with a liquid sending portion (not shown). The first end 512 of the circulation flow path 51, which is in communication with the inlet 901 of the reservoir 90, will also be referred to as the outlet 512.

The type of the liquid sending portion is not particularly limited, and examples thereof include liquid sending devices such as a roller pump and a pressure pump. The roller pump can feed the liquid in the flow path by, for example, pushing the flow path with a roller that rotates. In the case where a roller pump is used as the liquid sending portion, the contact region 514 of the circulation flow path 51 has, for example, a soft, preferably elastic, portion that comes into contact with the roller. As a specific example, as shown in FIG. 13, in the case of a configuration in which the recessed portion forming the contact region 514 in the substrate 311 is covered with the cover 313, for example, the cover 313 is preferably a soft cover. In this case, the roller is brought into contact with the contact region 514 of the circulation flow path 51 from the upper surface side of the fluid handling device 3 (i.e., the cover 313 side), and the roller is moved while being rotated in the direction of the arrow F. In the case where the roller moves while pressing the soft cover 313, the suction side (the first flow path 11 side) of the contact region 514 becomes depressed to suction the liquid, and the portion through which the roller passes becomes pressed to discharge the liquid. In this way, the liquid is suctioned and discharged in the circulation flow path 51, and the liquid can be circulated in the circulation route of the first flow path 11.

Also, in the case where a pressure pump is used, for example, the liquid may be fed by setting the inside of the flow path to a negative pressure to pull the liquid, or the liquid may be fed by setting the inside of the flow path to a positive pressure to push the liquid. In the case where a pressure pump is used as the liquid sending portion, for example, the pressure pump may be in communication with the contact region 514 of the circulation flow path 51 such that the inside of the circulation flow path 51 can be set to a negative pressure or a positive pressure. Specifically, for example, the inside of the contact region 514 of the circulation flow path 51 and the pressure pump are put in communication with each other.

A combination of the fluid handling device 3 and the liquid sending portion, specifically a combination in which the fluid handling device 3 is installed in the liquid sending portion, can be referred to as a fluid handling system.

The fluid handling system may, for example, further include a temperature control unit. For example, as described above, in the case where cells are arranged in the main flow path 10 and the first flow path 11, by controlling the temperature with the temperature control unit, the cells can be cultured or a temperature state close to that of a human body can be obtained.

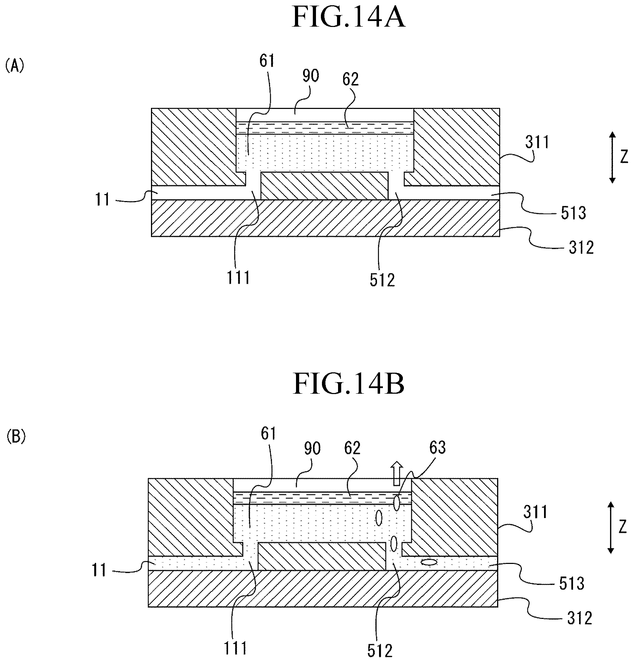

Next, a method for sending a liquid to the first flow path 11 in the fluid handling device 3 will be described below. FIG. 14 shows the state of the reservoir 90 during use. In FIG. 14, (A) and (B) are cross-sectional views of the reservoir similar to those in FIG. 13.

First, without driving the liquid sending portion, a liquid 61 to be sent to the first flow path 11 is supplied to the reservoir 90 using a pipette or the like. The liquid is not particularly limited, and any liquid can be used as desired, for example, an aqueous solvent such as water, a buffer solution, and a culture medium or the like; said aqueous solvent that includes a nutritional component, a blood component or the like; and blood or the like.

Subsequently, a non-affinity solvent 62 not having affinity for the liquid is further added to the reservoir 90 to which the liquid has been supplied. The non-affinity solvent may be, for example, a solvent that separates from the liquid and has a specific gravity lower than that of the liquid, one example being an oily solvent. Examples of the oily solvent include oils such as mineral oil. Due to adding the non-affinity solvent, as shown in FIG. 14(A), a non-affinity solvent layer 62 separates from the liquid layer 61 and is formed on the liquid layer 61. In the present embodiment, the non-affinity solvent layer 62 can be referred to, for example, as a solvent lid portion that separates from the liquid.

Then, the liquid sending portion is driven to start sending the liquid in the direction of the arrow F in FIG. 12. Accordingly, the liquid stored in the reservoir 90 is introduced from the reservoir 90 into the first flow path 11, passes through the first flow path 11, is introduced into the circulation flow path 51, passes through the circulation flow path 51, and returns to the reservoir 90, and this process is repeated.

According to the fluid handling device of the present embodiment, effects such as the following can be simultaneously achieved by the above-described configuration and method of use.

(1) Prevention of Entrance of Bubbles

When a liquid is introduced into a flow path, particularly a small flow path such as a micro flow path, if bubbles are present in the liquid, the bubbles may also be introduced into the flow path. In the case where the above-described blood vessel model is formed in the flow path, for example, the bubbles may physically collide with cells, which may result in detachment of the cells from the inner wall of the flow path, dissociation of the bond between the cells, and the like. In contrast, with the fluid handling device of the present embodiment, the reservoir 90 is in communication with the upstream side of the first flow path 11, thus making it possible to prevent bubbles from entering the first flow path 11.

That is to say, as shown in FIG. 14(B), when the liquid is introduced from the circulation flow path 51 into the reservoir 90, bubbles 63 may also enter. However, the bubbles 63 introduced into the reservoir 90 through the circulation flow path 51 move upward through the liquid layer 61, pass through the liquid layer 61 and the non-affinity solvent layer 62, and are released into the atmosphere. Thus, liquid that does not contain bubbles 63 is introduced from the liquid layer 61 stored in the reservoir 90 into the first flow path 11.

The positions of the through holes 901 and 902, for example, in the reservoir 90 are not limited to positions in the bottom portion 903, and may be positions in a side surface portion. In the case of positions in a side surface portion, for example, the side surface portion may be a portion above or below the upper surface of the liquid layer stored in the reservoir 90.

(2) Prevention of Contamination

In a fluid handling device having a flow path such as the above-described biofunctional chip, from the viewpoint of preventing contamination, it is desirable that the portion through which the liquid flows is a closed system as a whole. In the fluid handling device 3, as described above, the reservoir 90 is a tank that is open upward, and therefore the structure of the reservoir 90 itself is not a closed system but rather an open system. However, in the fluid handling device 3, as described above, the non-affinity solvent not having affinity for the liquid to be sent is added to the reservoir 90, and as such, the non-affinity solvent layer 62 covering the upper portion of the liquid layer 61 is formed inside the reservoir 90. Therefore, although the structure itself of the reservoir 90 is an open system, the liquid layer 61 in the reservoir 90 can realize a closed system during use. Therefore, according to the fluid handling device 3, the liquid layer 61 in the reservoir 90 can be prevented from coming into contact with the outside air, and contamination can be prevented.

In the fluid handling device 3 of the present embodiment, for example, the first flow path 11 and the circulation flow path 51 are closed systems, and the reservoir 90 connecting them is also a closed system due to the non-affinity solvent layer 62, and therefore, as a whole, the circulation route through which the liquid to be sent to the first flow path 11 flows can be said to be a closed system. Note that the main flow path 10 and the second flow path 12 can also be formed as a closed system by connecting the reservoir and the circulation flow path and forming the non-affinity solvent layer 62, as in the first flow path 11.

(3) Prevention of Evaporation of Liquid

As described above, in the fluid handling device 3, the liquid layer 61 in the reservoir 90 can be made to be a closed system by forming the non-affinity solvent layer 62 on the liquid layer 61. Therefore, according to the fluid handling device 3, evaporation of the liquid layer 61 in the reservoir 90 can be prevented.

(4) Avoidance of Influence of Air Expansion

As described in section (2) above, from the viewpoint of prevention of contamination, a fluid handling device such as the above-described biofunctional chip is desirably a closed system. On the other hand, in the biofunctional chip, for example, temperature control is performed by the temperature control unit for the purpose of culturing cells, reproducing a temperature state of the human body, and the like, as described above. The temperature range is not particularly limited, and for example, the temperature is controlled for each site according to the target process, in the range of 4 to 40°C. However, in the case of a fluid handling device having a closed system structure for the former purpose, when the temperature is changed for the latter purpose, the pressure inside (internal pressure of) the flow path may fluctuate due to air expansion. As a result, for example, there are cases where it is difficult to control the flow of the liquid, such as when the liquid flows unnecessarily in the flow path.

To address this, with the reservoir 90 of the fluid handling device 3, a closed system is not formed by the structure of the body, but rather, a closed system for the liquid layer 61 is achieved by forming the non-affinity solvent layer 62 on the liquid layer 61 inside the tank that is open upward as described above. The non-affinity solvent layer 62 is a solvent layer, and therefore has fluidity in the vertical direction. Therefore, for example, even if the internal pressure rises in any portion of the circulation route including the first flow path 11 in the fluid handling device 3, the closed system is maintained by the non-affinity solvent layer 62 in the reservoir 90, and furthermore, a rise in the internal pressure can be canceled out by rising of the liquid surface of the non-affinity solvent layer 62 that serves as a damper. As a result, it is possible to prevent the unnecessary flow of liquid in the flow path caused by fluctuation of the internal pressure as described above.

(5) Convenience of Sampling

As described in section (2) above, a fluid handling device such as the above-described biofunctional chip is desirably a closed system. Therefore, for example, in the case where the entire structure of the fluid handling device is a closed system, it is difficult to sample the liquid circulating inside. To address this, in the fluid handling device 3 of the present embodiment, the circulation route of the first flow path 11 is a closed system due to the formation of the non-affinity solvent layer 62 in the reservoir 90 as described above, but the structure of the reservoir 90 itself is an open system that is open upward. Therefore, for example, a sampling instrument such as a pipette can be inserted into the liquid layer 61 through the opening above the reservoir 90 to which the liquid that has passed through the first flow path 11 and the circulation flow path 51 returns, and the liquid in the reservoir 90 can be easily sampled by suction. Also, at the time of this sampling, the liquid layer 61 of the reservoir 90 is covered with the non-affinity solvent layer 62, and therefore, contact with the outside air is prevented, and contamination by the sampling can also be prevented. Note that sampling is preferably performed in a state where the driving of the liquid sending portion is stopped.

In the present embodiment, as shown in FIGS. 12 and 13, the contact region 514 of the circulation flow path 51 is formed by the recessed portion in the upper surface of the substrate 311, but the present invention is not limited to this, and the contact region 514 may be, for example, formed at the lower surface of the substrate 311. In this case, for example, a recessed portion forming the contact region 514 is formed in the lower surface of the substrate 311, and the cover 313 is disposed on the lower surface of the substrate 311 so as to cover the recessed portion. In the case where a roller pump is used as the liquid sending portion as described above, for example, the roller is brought into contact with the contact region 514 of the circulation flow path 51 from the lower surface side of the fluid handling device 3 (i.e., the lower surface side on which the cover 313 is disposed), and the roller is moved while being rotated in the direction of the arrow F.

(Eleventh Embodiment)

Although FIG. 12 illustrates a mode having one reservoir, the present invention is not limited to this, and the number of reservoirs can be appropriately set according to the intended use of the fluid handling device, and may be two or more.

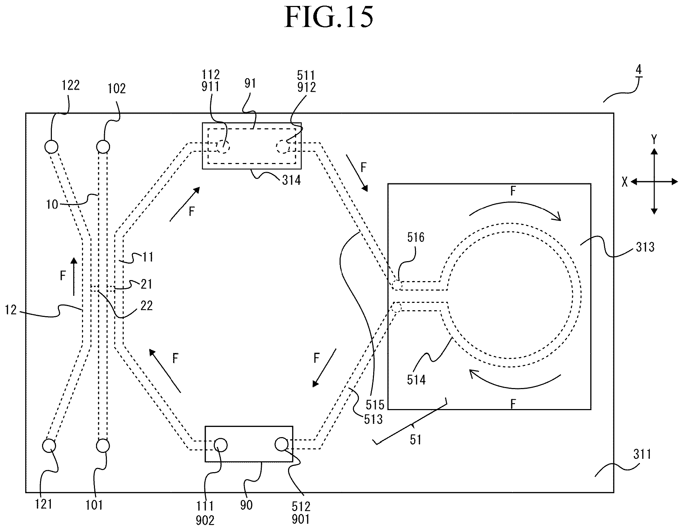

As an example of the fluid handling device of the present embodiment, FIG. 15 shows a mode having two reservoirs. FIG. 15 is a plan view of the fluid handling device as viewed from the upper surface side (top view), and with the exception of the fluid handling device having two reservoirs, the description of the tenth embodiment can be applied here. In FIG. 15, the portions that are the same as those in FIGS. 12 to 14 are denoted by the same reference numerals.

In a fluid handling device 4, a recessed portion that forms a reservoir 91 is further formed in one surface (the upper surface in FIG. 15, hereinafter called the "upper surface") of the substrate 311, and through holes that penetrate to the other surface (the lower surface in FIG. 15, hereinafter called the "lower surface") are further provided at two locations 911 and 912 in the bottom portion of the recessed portion that forms the reservoir 91. Also, a recessed portion that forms an introduction-side region 515 of the circulation flow path 51, which is an internal flow path, is further formed in the lower surface of the substrate 311. In the substrate 311, the through holes at the two ends 516 of the contact region 514 of the circulation flow path 51 are respectively in communication with the recessed portions forming the outlet-side region 513 and the introduction-side region 515 of the circulation flow path 51, and the two through holes 911 and 912 in the bottom portion of the reservoir 91 are respectively in communication with the recessed portion forming the first flow path 11 and the recessed portion forming the introduction-side region 515 of the circulation flow path 51.

The contact region 514 of the circulation flow path 51 on the upper surface side of the substrate 311 is connected, in the vertical direction, to the outlet-side region 513 and the introduction-side region 515 of the circulation flow path 51 on the lower surface side of the substrate 311 by the aforementioned through holes, and the reservoir 91 on the upper surface side of the substrate 311 is connected, in the vertical direction, to the first flow path 11 and the introduction-side region 515 of the circulation flow path 51 on the lower surface side of the substrate 311 by the through holes 911 and 912.

A cover 314 is further disposed on the upper surface of the substrate 311 so as to cover the recessed portion that forms the reservoir 91. The cover 314 is, for example, a resin cover, and may be hard, soft, or elastic, and the cover is preferably soft. The cover 314 is not particularly limited in terms of thickness, and may be, for example, a film or a sheet. The cover 314 is, for example, preferably a detachable cover.

With the fluid handling device 4 of the present embodiment, the liquid in the circulation route of the first flow path 11 can be circulated by driving the liquid sending portion, for example, as in the fluid handling device of the tenth embodiment. Specifically, in the present embodiment, for example, a liquid is introduced from the reservoir 90 into the first flow path 11, passes through the first flow path 11, is introduced into the reservoir 91, is introduced from the reservoir 91 into the introduction-side region 515 of the circulation flow path 51, passes through the contact region 514 and the outlet-side region 513 of the circulation flow path 51, and then returns to the reservoir 90, and this process is repeated.

The reservoir 91 in the present embodiment, for example, can be used for sampling the liquid circulated through the circulation route. Specifically, for example, at the time of sampling, the cover 314 covering the reservoir 91 is detached from the substrate 311, a pipette or the like is inserted, and as such, the liquid stored in the reservoir 91 can be sampled. After the sampling, for example, the reservoir 91 may be covered again by the cover 314.

In the case where the fluid handling device of the present invention has two or more reservoirs, the reservoir disposed between the outlet of the first flow path and the circulation flow path preferably has a non-solvent lid portion (e.g., a molded body such as a film) disposed at the opening of the reservoir such that the interior of the reservoir is airtight during use. On the other hand, it is preferable that the reservoir disposed between the inlet of the first flow path and the circulation flow path does not have a non-solvent lid portion at the time of use, and the liquid layer in the reservoir is a closed system due to the non-affinity solvent layer.

In the present application, in addition to the above-described fluid handling device of the present invention (first fluid handling device), another fluid handling device (hereinafter referred to as a second fluid handling device) will be described below.

[Second Fluid Handling Device]

As described above, a biofunctional chip is used as a pseudo model of a human body by, for example, causing blood or a substitute liquid to flow through the flow path. In order to use the biofunctional chip as a pseudo model, it is necessary to satisfy various environmental conditions under which the biofunctional chip becomes similar to those of a human body, but there is a problem that when the biofunctional chip has a structure in which various devices are combined in order to satisfy a plurality of environmental conditions, the biofunctional chip becomes difficult to handle.

Therefore, an object of the present invention is to provide a novel fluid handling device that integrally satisfies various environmental conditions similar to those of a human body.

A fluid handling device (second fluid handling device) according to an aspect of the present invention includes:

a body,
wherein the body includes a first flow path, a reservoir (also called "first reservoir" below) configured to store a liquid, and a circulation flow path,

the reservoir is a tank open upward,

the reservoir and the circulation flow path are in communication with each other, and

an inlet at a first end of the first flow path and an outlet at a second end of the first flow path are in communication with each other via the reservoir and the circulation flow path outside the first flow path.

According to the second fluid handling device of the present invention, for example, as an integrated device having the above-described configuration, effects such as the following (1) to (5) can be obtained. Note that regarding these effects, the descriptions of the tenth and eleventh embodiments of the first fluid handling device can be referred to.

(1) Prevention of Entrance of Bubbles

As described above, when a liquid is introduced into a flow path, particularly a small flow path such as a micro flow path, if bubbles are present in the liquid, the bubbles may also be introduced into the flow path. In the case where the above-described blood vessel model is formed in the flow path, for example, the bubbles may physically collide with cells, which may result in detachment of the cells from the inner wall of the flow path, dissociation of the bond between the cells, and the like.

However, in the second fluid handling device of the present invention, the outlet and the inlet of the first flow path are in communication with each other via the reservoir outside the first flow path. Therefore, for example, even in the case where the liquid flowing inside the fluid handling device contains bubbles, if the bubbles are introduced into the reservoir together with the liquid, the bubbles move upward, pass through the liquid layer stored in the reservoir, and are released into the atmosphere. Therefore, liquid not containing bubbles can be introduced from the liquid layer stored in the reservoir into the first flow path.

(2) Prevention of Contamination

In the second fluid handling device of the present invention, the reservoir is a tank that is open upward, and for example, at the time of use, a non-affinity solvent not having affinity for the liquid may be added to form a non-affinity solvent layer on the liquid phase stored in the reservoir. Therefore, according to the second fluid handling device of the present invention, a closed system for the liquid layer can be realized by forming the non-affinity solvent layer. In other words, according to the second fluid handling device, the liquid layer in the reservoir can be prevented from coming into contact with the outside air, and contamination can be prevented.

(3) Prevention of Evaporation of Liquid

In the second fluid handling device of the present invention, as described above, the non-affinity solvent layer can be formed on the liquid layer in the reservoir, and thus the liquid layer in the reservoir can be made a closed system. Therefore, according to the second fluid handling device, evaporation of the liquid layer in the reservoir can be prevented.

(4) Avoidance of Influence of Air Expansion

A fluid handling device such as the above-described biofunctional chip is desirably a closed system from the viewpoint of preventing contamination. On the other hand, in the biofunctional chip, for example, temperature control is performed by the temperature control unit for the purpose of culturing cells, reproducing a temperature state of the human body, and the like, as described above. However, in the case of a fluid handling device having a closed system structure for the former purpose, when the temperature is changed for the latter purpose, the pressure inside (internal pressure of) the flow path may fluctuate due to air expansion. As a result, for example, there are cases where it is difficult to control the flow of the liquid, such as when the liquid flows unnecessarily in the flow path.

In contrast, in the second fluid handling device of the present invention, a closed system is not formed by the structure of the body of the reservoir, but rather a closed system for the liquid layer is achieved by forming the non-affinity solvent layer on the liquid layer inside the tank that is open upward as described above. The non-affinity solvent layer is a solvent layer, and therefore has fluidity in the vertical direction. Therefore, according to the second fluid handling device of the present invention, for example, even if the internal pressure rises in any portion of the circulation route including the first flow path, the closed system is maintained by the non-affinity solvent layer in the reservoir, and furthermore, a rise in the internal pressure can be canceled out by rising of the liquid surface of the non-affinity solvent layer that serves as a damper. As a result, it is possible to prevent the unnecessary flow of liquid in the flow path caused by fluctuation of the internal pressure as described above.

(5) Convenience of Sampling

A fluid handling device such as the above-described biofunctional chip is desirably a closed system. Therefore, for example, in the case where the entire structure of the fluid handling device is a closed system, it is difficult to sample the liquid circulating inside. In contrast, in the second fluid handling device of the present invention, as described above, the circulation route of the first flow path is a closed system due to the formation of the non-affinity solvent layer in the reservoir, but the structure of the reservoir itself is an open system that is open upward. Therefore, for example, a sampling instrument such as a pipette can be inserted into the liquid layer through the opening above the reservoir storing the liquid from the first flow path, and the liquid in the reservoir can be easily sampled by suction. Also, at the time of this sampling, the liquid layer of the reservoir is covered with the non-affinity solvent layer, and therefore, contact with the outside air is prevented, and contamination by the sampling can also be prevented.

The second fluid handling device of the present invention is an integrated device due to the first flow path and the circulation flow path, which are internal flow paths, as well as the reservoir being formed in the main body as described above. Effects such as those described above can be achieved by such an integrated type of device.

In the second fluid handling device of the present invention, for example, the inlet of the first flow path may be in communication with the reservoir and the outlet of the first flow path may be in communication with the circulation flow path, or the outlet of the first flow path may be in communication with the reservoir and the inlet of the first flow path may be in communication with the circulation flow path, and the former is preferable.

In the second fluid handling device of the present invention, for example, the reservoir has through holes respectively on an upstream side and a downstream side relative to the flow direction of the liquid in the first flow path, the through hole on the upstream side is in communication with a first end of the circulation flow path, and the through hole on the downstream side is in communication with the inlet of the first flow path.

In the second fluid handling device of the present invention, for example, the two through holes are provided in a bottom portion of the first reservoir.

In the second fluid handling device of the present invention, for example, the first reservoir is a tank into which, at the time of use, a liquid that flows in the first flow path and a non-affinity solvent that does not have affinity for the liquid, are added.

In the second fluid handling device of the present invention, a second end of the circulation flow path may be directly in communication with the outlet of the first flow path or may be indirectly in communication with the outlet of the first flow path. In the latter case, the fluid handling device of the present invention may further include, for example, a second reservoir in addition to the first reservoir. In this case, in the fluid handling device of the present invention, for example, the second reservoir is a tank that is open upward, the second reservoir is in communication with a second end of the circulation flow path, and the second reservoir is in communication with the outlet of the first flow path.

In the second fluid handling device of the present invention, for example, the second reservoir has through holes respectively on an upstream side and a downstream side in a flow direction of the liquid in the first flow path, the through hole on the upstream side is in communication with the outlet of the first flow path, and the through hole on the downstream side is in communication with the first end of the circulation flow path.

In the second fluid handling device of the present invention, for example, the two through holes are provided in a bottom portion the second reservoir.

In the second fluid handling device of the present invention, for example, the second reservoir is a tank into which, at the time of use, a liquid that flows in the first flow path and a non-affinity solvent that does not have affinity for the liquid, are added.

In the second fluid handling device of the present invention, for example, the circulation flow path has an installation region for a liquid sending portion (also called "liquid sending device" below). The liquid sending portion is, for example, a roller pump or a pressure pump.

The second flow path handling device of the present invention may include, for example, the main flow path and a first connection flow path connecting the main flow path to the first flow path, as in the first fluid handling device, and may further include the second flow path and a second connection flow path connecting the main flow path to the second flow path.

Note that with the exception where conditions of the Laplace pressure are not limited, the description of the first fluid handling device of the present invention can be applied to the second fluid handling device of the present invention.

Also, a liquid introducing method of the present invention
uses the second fluid handling device of the present invention and includes:

a layer forming step of forming, in a reservoir of the fluid handling device, a liquid layer of a liquid to be introduced into the first flow path, and forming, on the liquid layer, a non-affinity solvent layer of a solvent not having affinity for the liquid; and

an introducing step of, after formation of the liquid layer and the non-affinity solvent layer, introducing the liquid in the liquid layer into the first flow path using a liquid sending portion.

In the introducing method of the present invention, for example, the layer forming step includes a step of supplying the liquid to the reservoir and a step of supplying the non-affinity solvent to the reservoir. In the present invention, the order in which the liquid and the non-affinity solvent are supplied to the reservoir is not particularly limited, and either one may be supplied first, or the two may be supplied simultaneously, and preferably, the non-affinity solvent is supplied after the liquid is supplied. As described above, for example, a solvent that separates from the liquid and has a specific gravity lower than that of the liquid can be used as the non-affinity solvent, and as such, the non-affinity solvent layer can be formed on the liquid layer regardless of the order in which the two are supplied.

The description of the tenth embodiment and the eleventh embodiment regarding the first fluid handling device, for example, can be applied to the introducing method of the present invention. Specifically, for example, the configurations of FIGS. 12 to 15 can be applied, with the exception where the conditions of the Laplace pressure are not limited.

Although the present invention has been described above with reference to embodiments, the present invention is not limited to the above-described embodiments. Various modifications that can be understood by those skilled in the art can be made to the configuration and details of the present invention within the scope of the present invention.

This application claims priority based on Japanese Patent Application No. 2022-13813 filed on January 31, 2022 and Japanese Patent Application No. 2022-106721 filed on June 30, 2022, the disclosures of which are incorporated herein in their entirety.

Industrial Applicability

As described above, according to the fluid handling device of the present invention, due to the pressure relationship of Condition 1 being satisfied, by introducing a fluid into the main flow path, the fluid can fill not only the main flow path but also the first connection flow path that connects the main flow path to the first flow path.

Description of Reference Numerals

1, 3, 4 Fluid handling device
10 Main flow path
11 First flow path
12 Second flow path
101, 111, 121, 511, 901, 911 Inlet
102, 112, 122, 512, 902, 912 Outlet
103, 113 Inlet flow path
104, 114 Outlet flow path
21, 71, 72, 73, 811, 812, 813 First connection flow path
211 End on first flow path side
22 Second connection flow path
30 Body
301 Upper substrate
302 Lower substrate
311 Substrate
312, 313, 314 Cover
40 Trap portion
50, 501, 502, 503 Branching portion
51 Circulation flow path
513 Outlet-side region
514 Contact region
515 Introduction -side region
60 Fluid A
61 Liquid layer
62 Non-affinity solvent layer
63 Bubble
601 Surface
80 Fluid B
81 First connection flow path group

82 Second connection flow path group
90, 91 Reservoir
903 Bottom portion
901, 902, 911, 912 Through hole

**Claims**

1. A fluid handling device comprising:

   a main flow path;
   a first flow path; and
   a first connection flow path connecting the main flow path to the first flow path,
   wherein the main flow path is a flow path into which a fluid A is introduced to fill the main flow path and the first connection flow path, and
   when the fluid A introduced into the main flow path flows from the main flow path into the first connection flow path, a Laplace pressure $P_{L1}$ acting at an interface between the fluid A and a fluid B filling the first connection flow path in advance satisfies Condition 1 below,

$$\text{Condition 1: } P_C > P_{L1},$$

   $P_C$: an internal pressure in a branching portion of the main flow path branching to the connection flow path, and
   $P_{L1}$: a Laplace pressure when a dynamic contact angle $\theta$ of the fluid A introduced from the main flow path into the connection flow path relative to an inner wall of the connection flow path is set to a value less than $\pi$ (pi).

2. The fluid handling device according to claim 1,

   wherein when the fluid A introduced into the main flow path flows from the main flow path into the first connection flow path, a Laplace pressure $P_{L2}$ acting at the interface between the fluid A and the fluid B filling the first connection flow path in advance satisfies Condition 2 below,

$$\text{Condition 2: } P_C < P_{L2},$$

   $P_C$: an internal pressure in the branching portion of the main flow path branching to the connection flow path, and
   $P_{L2}$: a Laplace pressure when the dynamic contact angle $\theta$ of the fluid A introduced from the main flow path into the connection flow path relative to the inner wall of the connection flow path is set to $\pi$.

3. The fluid handling device according to claim 1 or 2,
   wherein a cross-sectional area of a cross-section of the first connection flow path taken in a direction orthogonal to a flow direction of the fluid A at a position on a first flow path side of the first connection flow path is smaller than a cross-sectional area of a cross-section of the first connection flow path taken in the direction orthogonal to the flow direction of the fluid A at a position on a main flow path side of the first connection flow path.

4. The fluid handling device according to any one of claims 1 to 3,

   wherein a cross-section of the first connection flow path taken in a direction orthogonal to a flow direction of the fluid A is shaped as a quadrangle,
   the first connection flow path has a constant flow path depth, and
   a flow path width of the first connection flow path decreases from the main flow path side toward the first flow path side.

5. The fluid handling device according to any one of claims 1 to 3,

   wherein a cross-section of the first connection flow path taken in a direction orthogonal to a flow direction of the fluid A is shaped as a quadrangle,
   the first connection flow path has a constant flow path width, and

a flow path depth of the first connection flow path decreases from the main flow path side toward the first flow path side.

6. The fluid handling device according to any one of claims 1 to 3,

   wherein a cross-section of the first connection flow path taken in a direction orthogonal to a flow direction of the fluid A is shaped as a quadrangle, and
   a flow path depth and a flow path width of the first connection flow path both decrease from the main flow path side toward the first flow path side.

7. The fluid handling device according to any one of claims 1 to 6,
   wherein in the first connection flow path, the Laplace pressure $P_{L1}$ on the main flow path side is smaller than the Laplace pressure $P_{L1}$ on the first flow path side.

8. The fluid handling device according to any one of claims 1 to 7, comprising a plurality of the first connection flow paths.

9. The fluid handling device according to any one of claims 1 to 8,

   wherein the first flow path has an inlet for introduction of a fluid C and an outlet for discharge of the fluid C, and
   in the first flow path, a flow resistance from the branching portion, which branches to the first connection flow path, to the outlet is smaller than a flow resistance from the inlet to the branching portion.

10. The fluid handling device according to any one of claims 1 to 9,
    wherein the fluid A is a liquid or a sol.

11. The fluid handling device according to any one of claims 1 to 10,
    wherein the main flow path is a flow path for introduction of the fluid A comprising a cell mass.

12. The fluid handling device according to any one of claims 1 to 11,
    wherein the first flow path is a flow path for introduction of vascular endothelial cells for forming a blood vessel model.

13. The fluid handling device according to claim 12,
    wherein the vascular endothelial cells are HUVEC cells.

14. The fluid handling device according to any one of claims 1 to 13, further comprising:

    a second flow path; and
    a second connection flow path connecting the main flow path to the second flow path,
    wherein when the fluid A introduced into the main flow path flows from the main flow path into the second connection flow path, the Laplace pressure $P_{L1}$ acting at the interface between the fluid A and the fluid B filling the second connection flow path in advance satisfies Condition 1 below,

$$\text{Condition 1: } P_C > P_{L1},$$

    $P_C$: an internal pressure in the branching portion of the main flow path branching to the connection flow path, and
    $P_{L1}$: a Laplace pressure when the dynamic contact angle $\theta$ of the fluid A introduced from the main flow path into the connection flow path relative to the inner wall of the connection flow path is set to a value less than $\pi$.

15. The fluid handling device according to claim 14,

    wherein when the fluid A introduced into the main flow path flows from the main flow path into the second connection flow path, the Laplace pressure $P_{L2}$ acting at the interface between the fluid A and the fluid B filling the second connection flow path in advance satisfies Condition 2 below,

$$\text{Condition 2: } P_C < P_{L2},$$

$P_C$: an internal pressure in the branching portion of the main flow path branching to the connection flow path, and
$P_{L2}$: a Laplace pressure when the dynamic contact angle $\theta$ of the fluid A introduced from the main flow path into the connection flow path relative to the inner wall of the connection flow path is set to $\pi$.

16. The fluid handling device according to claim 14 or 15,

wherein the main flow path is a flow path for introduction of the fluid A comprising a cell mass, and
the second flow path is a flow path for introduction of a component involved in growth of the cell mass.

# FIG.1

# FIG.2

FIG.3

# FIG.4

# FIG.5

# FIG.6

$\theta < \pi$

$\theta = \pi$

# FIG.7

# FIG.8

# FIG.9

FIG.10

# FIG.11

104

503

$P_{C3}$

$P_{C3}$ ⇨

813

$P_{C2}$

502

$P_{C2}$ ⇨

812

$P_{C1}$

501

$P_{C1}$ ⇨

811

F

$P_{In}$ (kPa)

103

# FIG.12A

(A)

# FIG.12B

(B)

# FIG.13A

(A)

313

311

312

Z

# FIG.13B

(B)

514

514

516

313

311

312

12  10 11

Z

# FIG.13C

(C)

902  90  903  901

311

11

Z

513

312

111  512

# FIG.14A

(A)

# FIG.14B

(B)

# FIG.15

## INTERNATIONAL SEARCH REPORT

| | International application No. |
|---|---|
| | **PCT/JP2022/042865** |

**A. CLASSIFICATION OF SUBJECT MATTER**

*C12M 1/00*(2006.01)i; *B01J 19/00*(2006.01)i; *B81B 1/00*(2006.01)i; *G01N 37/00*(2006.01)i
FI: C12M1/00 A; B01J19/00 321; B81B1/00; G01N37/00 101

According to International Patent Classification (IPC) or to both national classification and IPC

**B. FIELDS SEARCHED**

Minimum documentation searched (classification system followed by classification symbols)

C12M1/00-3/10; G01N37/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | US 2017/0130187 A1 (THE REGENTS OF THE UNIVERSITY OF CALIFORNIA) 11 May 2017 (2017-05-11) abstract, claims, paragraphs [0047]-[0056], examples, fig. 1(B), 8 | 1-4, 7-8, 10-12, 14-16 |
| Y | | 1-16 |
| X | JP 2013-538582 A (MASSACHUSETTS INSTITUTE OF TECHNOLOGY) 17 October 2013 (2013-10-17) claims, paragraphs [0047]-[0074], [0109], examples, fig. 1, 3 | 1-4, 7-8, 10-16 |
| Y | | 1-16 |
| X | US 2019/0314814 A1 (REGENTS OF UNIVERSITY OF MINNESOTA) 17 October 2019 (2019-10-17) claims, examples | 1-4, 7, 10-11, 14-15 |
| A | | 5-6, 8-9, 12-13 |
| Y | JP 2014-530635 A (KOREA UNIVERSITY RESEARCH AND BUSINESS FOUNDATION) 20 November 2014 (2014-11-20) claims, fig. 2-5 | 1-16 |

☐ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **18 January 2023** | **31 January 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

| | International application No. |
|---|---|
| | **PCT/JP2022/042865** |

| Patent document cited in search report | | | Publication date (day/month/year) | Patent family member(s) | | | Publication date (day/month/year) |
|---|---|---|---|---|---|---|---|
| US | 2017/0130187 | A1 | 11 May 2017 | US | 2022/0025312 | A1 | |
| JP | 2013-538582 | A | 17 October 2013 | US | 2014/0057311 | A1 | |
| | | | | abstract, claims, paragraphs [0072]-[0099], [0134], examples, fig. 1, 3 | | | |
| | | | | WO | 2012/050981 | A1 | |
| | | | | EP | 2622342 | A1 | |
| | | | | CA | 2813211 | A1 | |
| | | | | SG | 189160 | A | |
| | | | | CN | 103477222 | A | |
| | | | | KR | 10-2013-0131326 | A | |
| | | | | SG | 10201508047S | A | |
| US | 2019/0314814 | A1 | 17 October 2019 | (Family: none) | | | |
| JP | 2014-530635 | A | 20 November 2014 | US | 2014/0302594 | A1 | |
| | | | | claims, fig. 2-5 | | | |
| | | | | JP | 2016-146851 | A | |
| | | | | WO | 2013/062383 | A1 | |
| | | | | EP | 2772530 | A1 | |
| | | | | KR | 10-2013-0046750 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022013813 A

- JP 2022106721 A

**Non-patent literature cited in the description**

- **IOANNIS K. ZERVANTONAKIS et al.** *PNAS*, 21 August 2012, vol. 109 (34), 13515-13520